(19) Europäisches Patentamt
European Patent Office
Office européen des brevets

(11) **EP 3 794 334 B1**

(12) **EUROPÄISCHE PATENTSCHRIFT**

(45) Veröffentlichungstag und Bekanntmachung des Hinweises auf die Patenterteilung:
**25.06.2025 Patentblatt 2025/26**

(21) Anmeldenummer: **19725663.9**

(22) Anmeldetag: **13.05.2019**

(51) Internationale Patentklassifikation (IPC):
*G01N 21/64* (2006.01)   *G01N 21/27* (2006.01)
*G01N 21/77* (2006.01)   *G01N 21/85* (2006.01)
*G01N 33/497* (2006.01)

(52) Gemeinsame Patentklassifikation (CPC):
**G01N 21/85; G01N 21/274; G01N 21/643;**
**G01N 21/645; G01N 21/77; G01N 33/497;**
G01N 21/6408; G01N 2021/6432; G01N 2021/6434;
G01N 2021/7763; G01N 2021/7786;
G01N 2021/7796; G01N 2021/8578;
G01N 2201/024; G01N 2201/1211

(86) Internationale Anmeldenummer:
**PCT/EP2019/062235**

(87) Internationale Veröffentlichungsnummer:
**WO 2019/219624 (21.11.2019 Gazette 2019/47)**

(54) **VERFAHREN ZUR DEGRADATIONSKOMPENSIERTEN AUSWERTUNG VON LUMINESZENZSENSOR-ERFASSUNGSSIGNALEN UND AUSWERTEVORRICHTUNG HIERFÜR**

METHOD FOR DEGRADATION-COMPENSATED EVALUATION OF LUMINESCENCE SENSOR DETECTION SIGNALS, AND EVALUATION APPARATUS THEREFOR

PROCÉDÉ D'ÉVALUATION COMPENSÉE PAR DEGRADATION DE SIGNAUX D'ACQUISITION DE CAPTEURS DE LUMINESCENCE ET UN TEL DISPOSITIF D'ÉVALUATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.05.2018 DE 102018207666**

(43) Veröffentlichungstag der Anmeldung:
**24.03.2021 Patentblatt 2021/12**

(73) Patentinhaber: **Hamilton Bonaduz AG**
**7402 Bonaduz (CH)**

(72) Erfinder:
• **SCHÖNFUSS, Dirk**
**7403 Rähzüns (CH)**
• **VERSCHININ, Valentin**
**8057 Zürich (CH)**

(74) Vertreter: **Ruttensperger Lachnit Trossin Gomoll Patent- und Rechtsanwälte PartG mbB Arnulfstraße 58 80335 München (DE)**

(56) Entgegenhaltungen:
GB-A- 2 496 657    US-A1- 2008 085 217
US-B1- 7 926 322

**Beschreibung**

Die vorliegende Erfindung betrifft ein Verfahren für eine degradationskompensierte Auswertung von Erfassungssignalen einer nach dem Prinzip der Lumineszenzlöschung arbeitenden Sensoranordnung. Die vorliegende Erfindung betrifft außerdem eine zur Durchführung eines solchen Verfahrens ausgebildete Auswertevorrichtung. Eine nach dem Prinzip der Lumineszenzlöschung arbeitende Sensoranordnung weist ein Luminophor, eine Anregungsstrahlungsquelle sowie wenigstens einen optischen Sensor auf. Das Luminophor strahlt als Reaktion auf eine Bestrahlung mit einer vorgegebenen modulierten Anregungsstrahlung eine Antwortstrahlung nach Maßgabe einer sensoranordnungstypischen Antwortcharakteristik ab, welche von dem wenigstens einen optischen Sensor erfasst wird. Die Antwortstrahlung hängt dabei nicht nur von der Anregungsstrahlung der Anregungsstrahlungsquelle ab, sondern auch vom Ausmaß einer Wechselwirkung des Luminophors mit einer Quencher-Substanz. Die Quencher-Substanz, etwa Sauerstoff, hat die Eigenschaft eine Lumineszenz des Luminophors zu löschen. Die durch die Anregungsstrahlung hervorgerufene Lumineszenz des Luminophors klingt daher in Gegenwart der mit dem Luminophor wechselwirkenden Quencher-Substanz schneller ab als dies bei Abwesenheit der Quencher-Substanz der Fall wäre. Die Antwortcharakteristik ist dabei der materialimmanente, vom Ausmaß der Wechselwirkung mit der Quencher-Substanz abhängige Zusammenhang zwischen Anregungsstrahlung und Antwortstrahlung des vorgegebenen Luminophors.

In der Regel werden derartige Sensoranordnungen messtechnisch eingesetzt, um die Anwesenheit einer bestimmten Quencher-Substanz nicht nur nachzuweisen, sondern zu quantifizieren. Ist beispielsweise Sauerstoff eine Quencher-Substanz für ein vorgegebenes Luminophor, kann man mit der Sensoranordnung den Sauerstoffgehalt bzw. die Sauerstoffkonzentration in einem zu prüfenden Prüffluid ermitteln. Hierzu muss das Prüffluid in lumineszenzlöschende Wechselwirkung mit dem Luminophor gebracht werden, im Falle einer Stoßlöschung beispielsweise in körperlichen Kontakt mit dem Luminophor.

Durch die von der Quencher-Substanz bewirkte Lumineszenzlöschung ändert sich abhängig von der Konzentration der Quencher-Substanz in dem Prüffluid ein Phasenwinkel einer Phasenverschiebung zwischen der modulierten Anregungsstrahlung und einer somit zwangsweise ebenfalls modulierten Antwortstrahlung. Mittels einer den Phasenwinkel zwischen Anregungs- und Antwortstrahlung berücksichtigenden Form der an sich bekannten Stern-Volmer-Gleichung kann man aus einem für eine bekannte Anregungsstrahlung ermittelten Phasenwinkel der Antwortstrahlung eine Konzentration der Quencher-Substanz bestimmen, etwa in Gestalt eines Partialdrucks der Quencher-Substanz im Prüffluid.

Durch die von der Quencher-Substanz bewirkte Lumineszenzlöschung ändert sich für eine vorgegebene Anregungsstrahlung abhängig von der Konzentration der Quencher-Substanz in dem Prüffluid eine Intensität der Antwortstrahlung. Mittels einer die Intensität der Antwortstrahlung berücksichtigenden Form der Stern-Volmer-Gleichung kann man aus einer für eine bekannte Anregungsstrahlung ermittelten Intensität der Antwortstrahlung ebenfalls eine Konzentration der Quencher-Substanz bestimmen.

Erfassungssignale der Sensoranordnung repräsentieren daher in der Regel entweder einen Phasenwinkel oder eine Intensität der Antwortstrahlung.

Aufgrund einer vor einem konkreten Erfassungsvorgang durchgeführten Kalibration der Sensoranordnung kann ganz allgemein einem Erfassungssignal, welches auf der vom optischen Sensor erfassten Antwortstrahlung beruht, ein Ergebniswert zugeordnet werden, welcher das gewünschte Ergebnis, beispielsweise eine Konzentration einer vorbestimmten Quencher-Substanz in einem Prüffluid, etwa den Sauerstoffpartialdruck in einem Prüffluid, repräsentiert. Somit wird bei der Kalibration der Sensoranordnung ganz allgemein ein Kalibrations-Wertezusammenhang ermittelt, welcher Werte des auf der Antwortstrahlung beruhenden Erfassungssignals mit Ergebniswerten verknüpft.

Ziel einer Auswertung von Erfassungssignalen einer nach dem Prinzip der Lumineszenzlöschung arbeitenden Sensoranordnung ist die Ermittlung des Ergebniswerts, auf den der Erfassungsvorgang abzielt.

Problematisch ist dabei, dass ein Luminophor mit der Zeit degradiert, etwa durch Ausbleichen, so genanntes "Bleaching". Mit fortschreitender Degradation des in einer Sensoranordnung verwendeten Luminophors ändert sich bei gleichbleibender Anregungsstrahlung und gleichbleibender Konzentration der Quencher-Substanz im Prüffluid die in Reaktion auf die Anregungsstrahlung abgestrahlte Antwortstrahlung des Luminophors. Dies bedeutet in der oben verwendeten Terminologie, dass sich die materialimmanente Antwortcharakteristik des Luminophors degradationsbedingt ändert.

In der vorliegenden Anmeldung ist die Nennung einer Degradation des Luminophors gleichbedeutend mit der Nennung einer Degradation der Sensoranordnung.

Mit fortschreitender Degradation des Luminophors vergrößert sich der Unterschied zwischen der aktuell zum jeweiligen Erfassungszeitpunkt herrschenden Antwortcharakteristik und der Bezugs-Antwortcharakteristik, welche während der Kalibration vorherrschte und auf welcher der immer noch gültige Kalibrations-Wertezusammenhang beruht. In der Folge weichen die Ergebniswerte, die anhand aktueller Erfassungssignale und basierend auf einer zeitlich zurückliegenden Kalibration bestimmt werden, zunehmend vom wahren Wert ab, im Falle eines Ausbleichens als sehr häufige Degradationsform beispielsweise hin zu zu niedrigen Erfassungswerten. Da wegen der Eigenschaft der Lumineszenzlöschung des Luminophors abnehmenden Erfassungswerten steigende Quencher-Substanz-Konzentrationen als Ergebniswerte zugeordnet sind, führt die Degradation des Luminophors in der Regel zur Ermittlung betragsmäßig zu hoher Ergebniswerte.

Die kalibrierte Sensoranordnung liefert bei bestimmungsgemäßer Verwendung zur Bestimmung von Ergebniswerten auf Grundlage der jeweils geltenden Antwortcharakteristik stets irgendeinen Erfassungswert und in der Folge auf Grundlage des jeweils geltenden Kalibrations-Wertezusammenhangs aus den Erfassungswerten irgendwelche Ergebniswerte. Für den Verwender der Sensoranordnung ist es im Stand der Technik nicht möglich zu erkennen, ob ein ermittelter hoher Ergebniswert das Resultat einer Degradation des Luminophors oder einer tatsächlich hohen Konzentration der Quencher-Substanz ist.

Ein Ansatz, degradationsbedingte Fehler in der Ermittlung von Ergebniswerten zu vermeiden, ist, die Sensoranordnung möglichst oft zu kalibrieren und somit die der Kalibration zugrunde liegende Bezugs-Antwortcharakteristik der sich durch Degradation des Luminophors ändernden aktuellen Antwortcharakteristik nachzuführen. Wegen der für die Kalibration aufzuwendenden Zeit senkt dies jedoch die Produktivität der Sensoranordnung ganz erheblich.

Ein anderer Ansatz liegt in der Korrektur der degradationsbedingt falschen Ergebniswerte. Ein Vorschlag hierzu ist aus der EP 2 887 054 A1 bekannt.

Diese Druckschrift lehrt, eine Degradation des Luminophors in der zur Auswertung des Sensoranordnungs-Erfassungssignals verwendeten Stern-Volmer-Gleichung korrigierend zu berücksichtigen. Dabei wird die Stern-Volmer-Gleichung in einer Form verwendet, die den erfassten Phasenwinkel als Argument verwendet.

Die EP 2 887 054 A1 schlägt vor, sowohl den Phasenbasiswert der Stern-Volmer-Gleichung, also derjenige Phasenwinkel, der bei Abwesenheit der Quencher-Substanz mit einem neuen, nicht degradierten Luminophor ermittelt wird, als auch die in der Gleichung verwendete Stern-Volmer-Konstante durch jeweilige Alterungsfaktoren zu modifizieren. Die Alterungsfaktoren sind wiederum abhängig von der Frequenz der Modulation der Anregungsstrahlung. Sie sind wegen ihrer Abhängigkeit von der Sensorstruktur sehr aufwendig für jeden Sensortyp und wegen ihrer Frequenzabhängigkeit für mehrere Frequenzen der Modulation der Anregungsstrahlung empirisch zu ermitteln.

Die gemäß der EP 2 887 054 A1 verwendeten Stern-Volmer-Gleichungen werden durch Exponentialfunktionen degradationskorrigiert. Dabei werden unterschiedliche Exponentialfunktionen als Korrektur-Faktoren mit dem Phasenbasiswert und mit der Stern-Volmer-Kostanten multipliziert. Die jeweiligen Alterungsfaktoren sind Teil des Exponenten der Exponentialfunktionen. Aufgrund der hieraus resultierenden Gleichungsstruktur ist zur Degradationskorrektur gemäß der EP 2 887 054 A1 bei jedem Erfassungsvorgang ein Gleichungssystem aufwendig numerisch zu lösen, was entweder die Bereitstellung einer Auswertevorrichtung mit ungewöhnlich hoher Rechenleistung erfordert oder, bei herkömmlicher Rechenleistung, den Erfassungsvorgang bis zum Erhalt des begehrten Ergebniswerts zeitlich verzögert. Außerdem ist die in der EP 2 887 054 A1 vorgeschlagene Degradationskorrektur abhängig von der Betriebsdauer des Sensors, was in der Realität nicht notwendigerweise der Fall ist.

Die Druckschrift GB 2 496 657 A offenbart den Gegenstand des Oberbegriffs des Anspruchs 1 sowie eine Vorrichtung, die dieses Verfahren ausführt; diese Druckschrift zitiert als Stand der Technik die Druckschriften GB 2 479 183 A, GB 2 380 790 A, WO 2007/066126 A1, WO 2004/077035 A1, DE 197 09 377 A1, JP 2010-133725 A, US 7,926,322 B1, US 5,030,420 A und US 2011/0184259 A1.

Die Druckschrift US 7,926,322 B1 offenbart den Gegenstand des Oberbegriffs des Anspruchs 1 sowie eine Vorrichtung, die dieses Verfahren ausführt; diese Druckschrift zitiert als Stand der Technik die Druckschriften US 5,060,505 A, US 5,887,048 A, US 6,205,272 B1, US 2006/0018045 A1 und US 5,030,420 A.

Die Druckschrift US 2008/0085217 A1 offenbart eine Luminiszenzsensoranordnung für eine Beatmungsvorrichtung zur luminiszenzbasierten Erfassung von Bestandteilen eines Messgases. Der bekannte Luminophor wird mit einer amplitudenmodulierten Anregungsstrahlung bestrahlt und strahlt folglich eine amplitudenmodulierte Antwortstrahlung ab. Auf Grundlage von Anregungsstrahlung und Antwortstrahlung wird ein Phasenunterschied zwischen diesen Strahlungen ermittelt, auf dessen Grundlage allein der Anteil des luminiszenzlöschenden Bestandteils im Messgas bestimmt wird.

Der Phasenunterschied wird nach Maßgabe einer ausschließlich zeitablaufbasiert bestimmten Degradation des Luminophors komponsiert.

Die Aufgabe der vorliegenden Erfindung ist daher, eine technische Lehre bereitzustellen, die es ermöglicht, den Einfluss einer Luminophordegradation auf die Auswertung von Erfassungssignalen einer nach dem Prinzip der Lumineszenzlöschung arbeitenden Sensoranordnung mit geringerem Aufwand als bisher und möglichst mit höherer Genauigkeit so weit als möglich zu eliminieren.

Diese Aufgabe wird erfindungsgemäß jeweils durch ein Verfahren nach Anspruch 1, eine Auswertevorrichtung nach Anspruch 6 und eine Messanordnung nach Anspruch 11 gelöst. Bevorzugte Ausführungsformen der Erfindung sind in den Unteransprüchen beschrieben.

Diese Aufgabe wird gemäß der vorliegenden Erfindung insbesondere gelöst durch ein Verfahren für eine degradationskompensierte Auswertung von Erfassungssignalen einer nach dem Prinzip der Lumineszenzlöschung arbeitenden Sensoranordnung, welche Sensoranordnung ein mit der Zeit degradierendes Luminophor, eine Anregungsstrahlungsquelle sowie wenigstens einen optischen Sensor aufweist. Das Luminophor strahlt als Reaktion auf eine Bestrahlung mit einer vorgegebenen modulierten Anregungsstrahlung in Abhängigkeit vom Ausmaß einer Wechselwirkung des Luminophors mit einer eine Lumineszenz des Luminophors löschenden Quencher-Substanz gemäß einer sensoranordnungstypischen Antwortcharakteristik eine von dem wenigstens einen optischen Sensor erfasste Antwortstrahlung ab. Die Sensoranordnung gibt als Erfassungssignale einen eine Intensität der Antwortstrahlung repräsentierenden erfassten Intensitätswert und einen einen Phasenunterschied der Antwortstrahlung bezüglich der Modulation der Anregungsstrahlung repräsentierenden erfassten Phasenwert aus. Für eine erfolgte Erfassung einer Antwortstrahlung wird eine betragsmäßige Abweichung eines der erfassten Werte aus erfasstem Intensitätswert und erfasstem Phasenwert, wobei die Abweichung auf einer degradationsbasierten Änderung der Antwortcharakteristik zur Zeit der erfolgten Erfassung bezüglich einer Bezugs-Antwortcharakteristik beruht, die einer Kalibration der Sensoranordnung zu Grunde liegt, nach Maßgabe sowohl des erfassten Intensitätswerts als auch des erfassten Phasenwerts betragsmäßig verringert. Somit wird ein degradationskompensierter erfasster Wert ermittelt. Ein auf die Quencher-Substanz bezogener Ergebniswert der erfolgten Erfassung wird dann nach Maßgabe eines vorbestimmten, unter Berücksichtigung der Bezugs-Antwortcharakteristik ermittelten Kalibrations-Wertezusammenhangs auf Grundlage des degradationskompensierten erfassten Werts bestimmt.

Anders als die EP 2 887 054 A1 zieht das Auswerteverfahren der vorliegenden Erfindung zur Kompensation der Luminophordegradation in äußerst vorteilhafterweise nicht nur entweder den erfassten Phasenwert oder den erfassten Intensitätswert heran, sondern zieht hierzu beide bei einer Erfassung erhältlichen Werte: Phasenwert und Intensitätswert, heran. Völlig überraschend wurde nämlich herausgefunden, dass für ein und dieselbe nach dem Prinzip der Lumineszenzlöschung arbeitende Sensoranordnung der Intensitätswert und der Phasenwert in jedem Degradationszustand jeweils in einem eindeutigen Zusammenhang stehen. Der Intensitätswert, dargestellt als Funktion des Phasenwerts, hat einen stetigen, hinreichenden und eindeutigen Graphen als Abbild. Selbstverständlich gilt dasselbe für den Phasenwert als Funktion des Intensitätswerts. Ein und derselben Sensoranordnung ist somit für eine vorgegebene Anregungsstrahlung und für unterschiedliche Konzentrationen an Quencher-Substanz in einem Prüffluid ein hinreichender und eindeutiger Zusammenhang zwischen dem Phasenwert und dem Intensitätswert einer Antwortstrahlung zugeordnet.

Dies gilt weiter überraschend unabhängig davon, ob sich die Konzentration der Quencher-Substanz in einem durch die Sensoranordnung zu überprüfenden Prüffluid oder beispielsweise die Temperatur des Prüffluids oder/und der Sensoranordnung ändert. Stets ist für eine auf Grundlage einer zum Kalibrationszeitpunkt herrschenden Bezugs-Antwortcharakteristik kalibrierte Sensoranordnung, vorzugsweise fabrikneue Sensoranordnung, für jede Konzentration der Quencher-Substanz im Prüffluid und für jede Temperatur des Prüffluids einem Phasenwert ein Intensitätswert eindeutig zugeordnet und umgekehrt. Durch Änderung der Konzentration der Quencher-Substanz oder/und durch Änderung der Temperatur des Prüffluids verlagern sich die dann bei einem Erfassungsvorgang erhaltenen Erfassungswerte nur längs des Zusammenhangs, verlassen diesen jedoch nicht. Nachfolgend ist dieser Zusammenhang zwischen Phasenwert und Intensitätswert für unterschiedliche Konzentrationen an Quencher-Substanz im Prüffluid und gegebenenfalls für unterschiedliche Prüffluid-Temperaturen nur kurz als Zusammenhang oder Phasenwert-Intensitätswert-Zusammenhang bezeichnet.

Dieser eindeutige Zusammenhang von Phasenwert und Intensitätswert für eine vorgegebene Antwortstrahlung und für ein Prüffluid mit unterschiedlichen Konzentrationen an Quencher-Substanz ist ein Beispiel für eine Antwortcharakteristik der Sensoranordnung bzw. eines bauart- und luminophorbedingt gleichen Typs von Sensoranordnungen. Wegen der Eindeutigkeit des Zusammenhangs ist es gleichgültig, ob der Intensitätswert als Funktion des Phasenwerts oder ob der Phasenwert als Funktion des Intensitätswerts angesehen wird. Wie weiter unten anhand des Ausführungsbeispiels zu erkennen ist, kann es praktischer sein, jenen Erfassungswert, der Eingangsgröße für die Anwendung des Kalibrations-Wertezusammenhangs zur Berechnung des Ergebniswertes ist, als Basis-Erfassungswert zu verwenden, und den jeweils anderen Erfassungswert als Funktion des Basis-Erfassungswerts aufzufassen bzw. zu verwenden.

Wie oben dargelegt wurde, ist es bei lediglich der Betrachtung nur eines erhaltenen Erfassungssignals bzw. -werts, also bei Betrachtung entweder nur des Phasenwerts oder nur des Intensitätswerts, unmöglich feststellbar, ob dieser Wert korrekt ist oder ob dieser Wert verfälscht ist, weil sich die aktuelle Antwortcharakteristik der Sensoranordnung aufgrund einer Degradation des Luminophors von der Bezugs-Antwortcharakteristik unterscheidet, auf der der immer noch verwendete Kalibrations-Wertezusammenhang der Sensoranordnung basiert. Dagegen ist es gemäß der vorliegenden Erfindung bei gleichzeitiger Betrachtung sowohl des Phasenwerts als auch des Intensitätswerts sehr wohl feststellbar, ob beispielsweise die Erfassung auf einem degradierten Luminophor oder auf einem der verwendeten Kalibration entsprechenden Luminophor basiert.

Für die Ermittlung des Ergebniswerts reicht nach wie vor einer der beiden erfassten Werte aus Phasenwert und Intensitätswert aus. Zur Feststellung, ob die Antwortcharakteristik des Luminophors der Sensoranordnung der der verwendeten Kalibration zugrundeliegenden Bezugs-Antwortcharakteristik ausreichend entspricht oder sich mittlerweile aufgrund von Degradation des Luminophors geändert hat, werden jedoch beide Werte benötigt. Liegt das Wertepaar aus Phasenwert und Intensitätswert außerhalb eines grundsätzlich frei, aber sinnvoll wählbaren vorgegebenen Toleranzbereichs um den bekannten Zusammenhang von Phasenwert und Intensitätswert der Sensoranordnung im Zustand ihrer letzten Kalibration (Kalibrationszustand), ist das Luminophor über die dem vorgegebenen Toleranzbereich zugrundeliegende Toleranzschwelle hinaus degradiert. Grafisch dargestellt liegt das Erfassungswertepaar aus Phasenwert und Intensitätswert in einem zweidimensionalen kartesischen Koordinatensystem, dessen Achsen einmal den Phasenwert und einmal den Intensitätswert repräsentieren, entfernt von der stetigen Kurve, die den Zusammenhang von Phasenwert und Intensitätswert im Kalibrationszustand der Sensoranordnung für unterschiedliche Konzentrationen an Quencher-Substanz im Prüffluid repräsentiert. Der Kalibrationszustand ist dabei der Zustand der Sensoranordnung zum Zeitpunkt der letzten, also aktuell noch verwendeten Kalibration.

Weiter völlig überraschend wurde festgestellt, dass der Zusammenhang von Phasenwert und Intensitätswert einer Sensoranordnung auch in einem vom Kalibrationszustand abweichenden Degradationszustand eindeutig und hinreichend ist, nur eben verschieden vom Phasenwert-Intensitätswert-Zusammenhang des Kalibrationszustands. Grafisch betrachtet liegen die Erfassungswertepaare von Phasenwert und Intensitätswert einer Sensoranordnung für unterschiedliche Konzentrationen der Quencher-Substanz im Prüffluid oder/und für unterschiedliche Temperaturen des Prüffluids in einem kartesischen Koordinatensystem mit den genannten Achsen für den Kalibrationszustand und für jeden Degradationszustand jeweils auf einer eindeutigen stetigen Kurve oder/und sind durch eine jeweils eindeutige Gleichung beschreibbar, wobei die einzelnen Kurven der unterschiedlichen Degradationszustände eine zueinander ähnliche Gestalt bzw. einen zueinander ähnlichen Verlauf aufweisen, jedoch zueinander verschoben oder/und geneigt sind.

Sind für eine vorgegebene Sensoranordnung bzw. einen vorgegebenen Typ einer Sensoranordnung die jeweiligen Zusammenhänge von Phasenwerten und Intensitätswert für unterschiedliche Degradationszustände bekannt, kann aus einem bei einer Erfassung erhaltenen Wertepaar aus Phasenwert und Intensitätswert der Degradationszustand - gegebenenfalls unter Anwendung von Extrapolation oder Interpolation - ermittelt und hieraus das entsprechende theoretische Wertepaar aus Phasenwert und Intensitätswert ermittelt werden, welches unter ansonsten gleichen Erfassungsbedingungen im Kalibrationszustand als Erfassungssignale (Erfassungswertepaar) ausgegeben worden wäre. Statt eines Wertepaares kann nur ein Wert aus Phasenwert und Intensitätswert dieses theoretischen Wertepaares ausgegeben werden. Dieser Wert: Phasenwert oder Intensitätswert, des theoretischen Wertepaares ist folglich ein degradationskompensierter Wert, anhand dessen mit dem bereits vorhandenen Kalibrations-Wertezusammenhang der eigentlich gewünschte Ergebniswert ermittelt werden kann.

Die Zusammenhänge von Intensitätswert und Phasenwert für unterschiedliche Konzentrationen der Quencher-Substanz im Prüffluid oder/und für unterschiedliche Temperaturen des Prüffluids in unterschiedlichen Degradationszuständen von fabrikneu/undegradiert bis vollständig degradiert können an einer oder mehreren Sensoranordnungen eines gleichen Typs von Sensoranordnungen empirisch festgestellt und dann in einem Datenspeicher einer Auswertevorrichtung für diesen Typ von Sensoranordnung hinterlegt werden. Grundsätzlich kann dann eine einzige Kalibration der Sensoranordnung, etwa zu Beginn ihrer Inbetriebnahme, ausreichen, weil spätere Erfassungsergebnisse in Degradationszuständen über die bekannten empirisch ermittelten Zusammenhänge, gegebenenfalls unter Zuhilfenahme von Extrapolation oder Interpolation, auf den Kalibrationszustand zurückgeführt werden können. Nach einer solchen Ermittlung des degradationskompensierten erfassten Werts kann anhand des aktuell verwendeten Kalibrations-Wertezusammenhangs ein ausreichend korrekter Ergebniswert ermittelt werden.

Ausgehend von den empirisch ermittelten Wertepaar-Zusammenhängen kann zur Erleichterung der Ermittlung des degradationskompensierten erfassten Werts ein Funktions- oder Gleichungssystem aus den Wertepaar-Zusammenhängen entwickelt werden, das die rechnertechnische Ermittlung des degradationskompensierten erfassten Werts erleichtert. Beispielsweise können über mehrere Intensitätswert-Phasenwert-Zusammenhänge des Kalibrationszustands und der einzelnen Degradationszutände hinweg verlaufende Iso-Konzentrationszusammenhänge bzw. -funktionen oder -spuren bzw. -verläufe bestimmt werden, die für eine vorgegebene Temperatur des Prüffluids jene Wertepaare aus Intensitätswert und Phasenwert verbinden, die jeweils der gleichen Konzentration der Quencher-Substanz bei unterschiedlichen Degradationszuständen des Sensoranordnungstyps zugeordnet sind. Diese können für Konzentrationen der Quencher-Substanz in Sprüngen vorbestimmter Sprungweite, etwa bei Angabe der Konzentration in vol.-% oder Gew.-% alle 10 Prozentpunkte, experimentell bestimmt werden. Diese Funktionen können ebenfalls in einem Datenspeicher einer Auswertevorrichtung hinterlegt werden. Zwischenwerte von Konzentrationswerten, für die keine Funktionen ermittelt wurden, werden durch Extra- oder Interpolation errechnet.

Eine solche Funktions- oder Gleichungsschar kann wiederum für mehrere relevante Prüffluid-Temperaturen ermittelt werden, sodass auch ein degradationskompensierter erfasster Wert aus Erfassungssignalen für Erfassungsvorgänge mit unterschiedlichen Prüffluidtemperaturen korrekt berechnet werden kann. Erfassungssignale von Erfassungsvorgängen, die bei von diesen Prüffluid-Temperaturen abweichenden Prüffluid-Temperaturen erzielt wurden, können durch Extra- oder Interpolation verarbeitet werden.

Derartige Funktions- oder Gleichungssysteme oder Funktions- oder Gleichungssystemscharen bilden ganz allgemein einen Kompensations-Wertezusammenhang, welcher erfasste Intensitäts- und Phasenwerte mit einem degradationskompensierten erfassten Wert verknüpft. Dieser Kompensations-Wertezusammenhang kann graphisch als Kurven oder Kurvenscharen, analytisch als Gleichungssystem oder Gleichungssystemschar oder in Tabellenform vorliegen und in einem Datenspeicher gespeichert sein. Analytische Darstellungen des Kompensations-Wertezusammenhangs können durch an sich bekanntes Fitting von Gleichungen an durch experimentelle Messungen oder/und theoretische Überlegungen erhaltene Punktescharen erhalten werden, beispielsweise im Wege eines Verfahrens der kleinsten Fehlerquadrate.

Ein Kompensations-Wertezusammenhang kann auch nur durch eine einzige Funktion oder Kurve oder Tabelle bereitgestellt sein, wie weiter unten gezeigt werden wird.

Daher umfasst das erfindungsgemäße Verfahren gemäß einer Weiterbildung der vorliegenden Erfindung den Schritt, den degradationskompensierten erfassten Wert auf Grundlage eines vorbestimmten Kompensations-Wertezusammenhangs mit Eingangsgrößen auf Grundlage des erfassten Intensitätswerts und des erfassten Phasenwerts zu ermitteln. Da zur Ermittlung des Ergebniswerts ein erfasster Wert ausreicht, wird für einen Erfassungsvorgang bevorzugt nur genau ein erfasster Wert aus erfasstem Intensitätswert und erfasstem Phasenwert auf Grundlage eines vorbestimmten Kompensations-Wertezusammenhangs auf Grundlage sowohl des erfassten Intensitätswerts als auch des erfassten Phasenwerts degradationskompensiert.

Das obige Verfahren kann jedoch in äußerst vorteilhafter Weise vereinfacht werden, indem man die oben beschriebenen Zusammenhänge zwischen Phasenwert und Intensitätswert für unterschiedliche Konzentrationen an Quencher-Substanz in einem Prüffluid bei vorgegebener Anregungsstrahlung, und damit die Antwortcharatkteristik, nicht in einem dimensionsbehafteten Raum, sondern in einem dimensionslosen Raum betrachtet und zumindest für einen Teil der Auswertung verwendet. Es hat sich nämlich gezeigt, dass die Funktionen der Zusammenhänge von Phasenwert und Intensitätswert für ein und denselben Sensoranordnungstyp im Kalibrationszustand stets denselben dimensionslosen Phasenwert-Intensitätswert-Zusammenhang bilden. Im dimensionsbehafteten Raum kann zwar für Sensoranordnungen ein und desselben Sensoranordnungstyps stets durch Kalibration sichergestellt werden, dass die Antwortcharakteristik einer Sensoranordnung für einen Erfassungsvorgang der Bezugs-Antwortcharakteristik entspricht. Abhängig vom Degradationszustand der Sensoranordnung unterscheiden sich die einzelnen, den unterschiedlichen Degradationszuständen zugeordneten Bezugs-Antwortcharakteristika voneinander.

Völlig überraschend hat sich gezeigt, dass unabhängig vom physischen Degradationszustand einer Sensoranordnung deren Bezugs-Antwortcharakteristika in dimensionsloser Notation immer die gleichen sind. Durch Degradation des Luminophors seit der letzten Kalibration unterscheidet sich zwar auch bei dimensionsloser Notation zunehmend die aktuelle Antwortcharakteristik von der Bezugs-Antwortcharakteristik, die der Erstellung des immer noch verwendeten Kalibrations-Wertezusammenhangs zugrunde lag. Eine erneute Kalibration der Sensoranordnung führt jedoch in dimensionsloser Notation nicht zu einer neuen dimensionslosen Bezugs-Antwortcharakteristik, - wie dies in dimensionsbehafteter Notation der Fall wäre - sondern führt zurück zu der bereits bekannten dimensionslosen Bezugs-Antwortcharakteristik. Dies vereinfacht den zur Degradationskompensation notwendigen Aufwand an Rechenleistung und Rechnerinfrastruktur enorm.

Dieser Umstand ermöglicht in besonders vorteilhafter Weise, dass eine einzige Gleichung bzw. Funktion als Kompensations-Wertezusammenhang zur Ermittlung des degradationskompensierten Erfassungswerts ausreicht.

Dieser Effekt beruht physikalisch-rechentheoretisch auf der Tatsache, dass sich zwar mit der Degradation des Luminophors dessen Performanz ändern, nicht jedoch die dieser Performanz zugrundeliegenden technischen Vorgänge und Wirkungen. Die vom Degradationszustand abhängigen Unterschiede können gleichsam "herausgekürzt" werden, wenn sowohl der erfasste Intensitätswert als auch der erfasste Phasenwert unter Verwendung von wenigstens einem die Sensoranordnung charakterisierenden Systemparameter oder/und von wenigstens einem dem Erfassungsprozess entstammenden Prozessparameter in einen dimensionslosen erfassten Intensitätswert bzw. in einen dimensionslosen erfassten Phasenwert transformiert werden. Eine solche Transformation ist zwar nicht zwingend notwendig, um die Vorteile der vorliegenden Erfindung zu erzielen. Die Vorteile lassen sich in dimensionsloser Notation der Erfassungsvorgänge jedoch schneller, mit geringerem Aufwand an Rechnerinfrastruktur und mit höherer Genauigkeit erzielen.

Ähnlichkeitstheoretisch bedeutet der Übergang der Betrachtung und datentechnischen Verarbeitung eines technischen Vorgangs von einer dimensionsbehafteten zu einer dimensionslosen Notation den Übergang von einem dem notierten Prozess fremden Standard-Normalensystem, wie etwa dem System der SI-Einheiten, zu einem prozesseigenen Koordinaten- oder Normalensystem. Dies ist etwa aus der Strömungsmechanik bekannt. So verhalten sich Strömungen mit demselben Betrag der dimensionslosen Reynoldszahl hinsichtlich bestimmter strömungsmechanischer Effekte völlig identisch, unabhängig vom strömenden Medium, der konkreten Strömungsgeschwindigkeit und den Abmessungen der Strömung. In dimensionsbehafteter Notation, etwa im SI-Einheitensystem, würden diese Strömungen zwar durch gleiche Grundgleichungen, jedoch mit völlig verschiedenen Parameterwerten beschrieben werden. In systemeigener dimensionsloser Notation werden sie alle durch denselben betragsgleichen Wert beschrieben.

Ähnliches gilt vorliegend für die Sensoranordnungen gleichen Typs, wobei hier die Bildung weitaus weniger komplizierter dimensionsloser Größen ausreicht. Beispielsweise kann jeder erfasste Phasenwert mittels Division durch einen durch Versuch vorbestimmten und quantifizierten Bezugs-Phasenwert in einen dimensionslosen Phasenwert überführt werden. Der vorbestimmte und quantifizierte Bezugs-Phasenwert kann beispielsweise der Phasenwert sein, den die Sensoranordnung bei einer vorbestimmten Quencher-Konzentration im Prüffluid liefert, etwa bei einem Quencher-Anteil von 50 vol.-% oder Gew.-% oder bei einem vorbestimmten Quencher-Partialdruck. Entsprechendes gilt mutatis mutandis für die Transformation des erfassten Intensitätswerts, wobei bevorzugt die der Bezugs-Phasenwert und der Bezugs-Intensitätswert bei derselben Quencher-Konzentration bestimmt werden.

Zur besseren Vergleichbarkeit und damit zur erleichterten datentechnischen Verarbeitbarkeit der erfassten Werte: Phasenwert und Intensitätswert, werden diese bevorzugt in einen dimensionslosen normierten erfassten Intensitätswert und dimensionslosen normierten erfassten Phasenwert transformiert. Dies kann dadurch erreicht werden, dass von dem jeweiligen erfassten Wert zunächst ein vorbestimmter Extremwert subtrahiert wird, etwa der Erfassungswert bei maximaler Konzentration der Quencher-Substanz, also bei Verwendung der reinen Quencher-Substanz als Prüffluid. Bei nach dem Prinzip der Lumineszenzlöschung arbeitenden Sensoranordnungen ist dies in der Regel ein betragsmäßig minimaler Erfassungswert. Der so erhaltene Differenzbetrag wird anschließend dividiert durch den Betrag des maximal möglichen erhaltbaren Erfassungswertebereichs der Sensoranordnung bzw. des Sensoranordnungstyps. Dieser Bereich ist wiederum ein Differenzbetrag und wird ermittelt durch die Bildung der Differenz zwischen dem, in der Regel betragsmäßig maximalen, Erfassungswert bei minimaler Konzentration der Quencher-Substanz, also bei Verwendung des von der Quencher-Substanz völlig freien Prüffluids, und dem Erfassungswert bei maximaler Konzentration der Quencher-Substanz. Diese letztgenannten Differenzenbeträge, die den jeweiligen maximal möglichen Wertebereich des jeweiligen Erfassungswerts beschreiben, sind dann für den Phasenwert als Erfassungswert der oben genannte Bezugs-Phasenwert und für den Intensitätswert als Erfassungswert der Bezugs-Intensitätswert.

Die Bestimmung Bezugs-Phasenwert und Bezugs-Intensitätswert erfolgt dabei bevorzugt unmittelbar nach der Kalibration, um sicherzustellen, dass die aktuelle Antwortcharakteristik der Sensoranordnung mit der der Kalibration zugrunde liegenden Bezugs-Antwortcharakteristik übereinstimmt.

Im Falle der dimensionslosen Normierung der Erfassungssignale weisen diese stets Beträge auf, die zwischen 0 und 1 liegen.

Dann ist der vorbestimmte Kompensations-Wertezusammenhang bevorzugt ein vorbestimmter dimensionsloser Kompensations-Wertezusammenhang, so dass der dimensionslose normierte Erfassungswert unmittelbar und ohne vorherige Rückumwandlung in einen dimensionsbehafteten Wert bezüglich einer eventuellen Degradation des Luminophors kompensiert werden kann. Nach Maßgabe des dimensionslosen Kompensations-Wertezusammenhangs kann der degradationskompensierte erfasste Wert mit Eingangsgrößen auf Grundlage des dimensionslosen, bevorzugt normierten, erfassten Intensitätswerts und des dimensionslosen, bevorzugt normierten, erfassten Phasenwerts, ermittelt werden. Der degradationskompensierte Erfassungswert ist dann bevorzugt ebenfalls ein dimensionsloser, besonders bevorzugt ein dimensionsloser normierter degradationskompensierter erfasster Wert bzw. Erfassungswert.

Wenn der degradationskompensierte erfasste Wert ein dimensionsloser, bevorzugt ein dimensionsloser normierter, degradationskompensierter erfasster Wert ist, kann dieser entweder in einen dimensionsbehafteten degradationskompensierten erfassten Wert umgerechnet und mit einer Eingangsgröße auf Grundlage desselben dann mit dem vorbestimmten Kalibrations-Wertezusammenhang der Ergebniswert bestimmt werden. Oder der vorbestimmte Kalibrations-Wertezusammenhang ist dazu ausgebildet unmittelbar mit einer Eingangsgröße auf Grundlage des dimensionslosen, bevorzugt dimensionslosen normierten, degradationskompensierten erfassten Werts den Ergebniswert zu bestimmen. Hierzu kann, aber muss nicht, der vorbestimmte Kalibrations-Wertezusammenhang ein dimensionsloser, bevorzugt ein dimensionsloser normierter, vorbestimmter Kalibrations-Wertezusammenhang sein.

Der Begriff "Eingangsgröße auf Grundlage eines Werts" schließt sowohl den Wert selbst als auch einen Funktionswert einer Funktion ein, dessen Argument der Wert ist.

Nicht nur sind die dimensionlosen Bezugs-Antwortcharakteristika ein und derselben Sensoranordnung oder ein und desselben Sensoranordnungstyps unabhängig vom Degradationszustand der Sensoranordnung identisch. Auch die dimensionslosen Antwortcharaktistika ein und derselben Sensoranordnung oder ein und desselben Sensoranordnungstyps sind für denselben Degradationszustand bezüglich des Kalibrationszustands des aktuell verwendeten Kalibrations-Wertebereich in einem weiten Bereich der nutzbaren Betriebslebensdauer einer Sensoranordnung identisch. Daher kann mit Hilfe eines dimensionslosen Kompensations-Wertezusammenhangs eine Degradationskompensation des Erfassungswerts erfolgen, ohne dass zuvor überhaupt bestimmt werden muss, ob die Sensoranordnung degradiert ist oder nicht. Der dimensionslose Kompensations-Wertezusammenhang wird schlichtweg auf das aktuell erhaltene dimensionslose Erfassungswertepaar angewendet. Es reicht aus, dass der dimensionslose Kompensations-Wertezusammenhang einen Erfassungswert des Erfassungswertepaars auf die bekannte degradationsinvariante dimensionslose Bezugs-Antwortcharakteristik zurückführt. Der dimensionslose Kompensations-Wertezusammenhang kann daher eine Abbildungsvorschrift sein, die lediglich einen der beiden erfassten Werte, welche beide aufgrund von Alterung bzw. Degradation des Luminophors außerhalb des die Bezugs-Antwortcharakteristik repräsentierenden degradationsinvarianten Zusammenhangs von dimensionslosem Intensitätswert und dimensionslosem Phasenwert liegen, auf Grundlage beider erfasster Werte auf den bekannten degradationsinvarianten Phasenwert-Intensitätswert-Zusammenhang überführt.

Grundsätzlich kann der degradationskompensierte erfasste Wert entweder ein degradationskompensierter erfasster Phasenwert oder ein degradationskompensierter erfasster Intensitätswert sein. Da nach bisheriger Erfahrung der durch Modulation der Anregungsstrahlung provozierbare Phasenwert bei seiner Auswertung den Ergebniswert mit der höheren Genauigkeit liefert, ist bevorzugt der degradationskompensierte erfasste Wert der degradationskompensierte erfasste Phasenwert ist. Dies gilt unabhängig von der Notation des erfassten Werts in einem dimensionsbehafteten, einem dimensionslosen oder einem normierten Werteraum.

Die oben genannte Aufgabe wird außerdem gelöst durch eine Auswertevorrichtung, welche zur Ausführung des oben beschriebenen Verfahrens ausgebildet ist. Die Auswertevorrichtung ist somit ausgebildet zur degradationskompensierten Auswertung von Erfassungssignalen einer nach dem Prinzip der Lumineszenzlöschung arbeitenden Sensoranordnung mit einem mit der Zeit degradierenden Luminophor, mit einer Anregungsstrahlungsquelle sowie mit wenigstens einem optischen Sensor, wobei das Luminophor als Reaktion auf eine Bestrahlung mit einer vorgegebenen modulierten Anregungsstrahlung in Abhängigkeit vom Ausmaß einer Wechselwirkung des Luminophors mit einer eine Lumineszenz des Luminophors löschenden Quencher-Substanz gemäß einer sensoranordnungstypischen Antwortcharakteristik eine von dem wenigstens einen optischen Sensor erfasste Antwortstrahlung abstrahlt, wobei die Auswertevorrichtung einen Dateneingangskanal aufweist, welcher dazu ausgebildet ist, von der Sensoranordnung als Erfassungssignale den eine Intensität der Antwortstrahlung repräsentierenden erfassten Intensitätswert und den einen Phasenunterschied der Antwortstrahlung bezüglich der Modulation der Anregungsstrahlung repräsentierenden erfassten Phasenwert an eine Datenverarbeitungseinheit der Auswertevorrichtung zu übertragen, wobei die Datenverarbeitungseinheit einen Datenspeicher zur Speicherung von Daten und eine Recheneinheit zur Verarbeitung von Daten aufweist, wobei in dem Datenspeicher wenigstens der vorbestimmte, unter Berücksichtigung der Bezugs-Antwortcharakteristik ermittelte Kalibrations-Wertezusammenhang gespeichert ist, wobei die Auswertevorrichtung dazu ausgebildet ist, den degradationskompensierten erfassten Wert aus erfasstem Intensitätswert und erfasstem Phasenwert nach Maßgabe sowohl des erfassten Intensitätswerts als auch des erfassten Phasenwerts zu ermitteln und den auf die Quencher-Substanz bezogenen Ergebniswert der erfolgten Erfassung nach Maßgabe des Kalibrations-Wertezusammenhangs auf Grundlage des degradationskompensierten erfassten Werts zu bestimmen und auszugeben.

Die Recheneinheit kann durch einen Mikrocomputer, einen integrierten Schaltkreis und dergleichen realisiert sein. Die Auswertevorrichtung kann ein Computer mit einem darauf ablaufbaren Datenverarbeitungsprogramm sein, das in einem mit der Recheneinheit in Datenübertragungsverbindung stehenden Datenspeicher gespeichert ist. Die obigen vorteilhaften Weiterbildungen des Verfahrens sind auch Weiterbildungen der zur Durchführung des Verfahrens Auswertevorrichtung.

Die Auswertevorrichtung kann gemäß den Ausführungen zum erfindungsgemäßen Verfahren zusätzlich oder alternativ dazu ausgebildet sein, aus einem Vergleich eines einem Erfassungsvorgang zugeordneten Paars von Erfassungssignalen mit der im Datenspeicher hinterlegten Bezugs-Antwortcharakteristik, auf welcher der aktuell verwendete Kalibrations-Wertezusammenhang beruht, zu ermitteln, ob das Luminophor der Sensoranordnung über eine vordefinierte Toleranzschwelle hinaus degradiert ist. Dies ist immer dann der Fall, wenn das Paar von Erfassungssignalen in der Koordinatenebene, die definiert ist durch Phasenwert und Intensitätswert, außerhalb eines vorgegebenen Toleranzbands um den Phasenwert-Intensitätswert-Zusammenhang gelegen ist, welcher die Bezugs-Antwortcharakteristik repräsentiert. Die Toleranzschwelle kann 0 sein, ist jedoch bevorzugt betragsmäßig von 0 verschieden, da der Phasenwert-Intensitätswert-Zusammenhang ein zwischen einzelnen Stützwerten interpolierter Zusammenhang ist und im Interpolationsbereich selbst nur einen Näherungs-Zusammenhang liefert. Bevorzugt gibt die Auswertevorrichtung einen Warnhinweis aus, wenn sie eine über die vorbestimmte Toleranzschwelle hinaus reichende Degradation des Luminophors ermittelt. Die Ermittlung einer nicht mehr tolerierten Degradation kann auf der Verarbeitung dimensionsbehafteter oder/und dimensionsloser oder/und normierter Werte als Daten beruhen.

Gemäß einer bevorzugten Weiterbildung der Auswertevorrichtung ist in dem Datenspeicher auch der vorbestimmte Kompensations-Wertezusammenhang gespeichert. Die Recheneinheit ist bevorzugt dazu ausgebildet, den degradationskompensierten erfassten Wert nach Maßgabe des vorbestimmten Kompensations-Wertezusammenhangs mit Eingangsgrößen auf Grundlage des erfassten Intensitätswerts und des erfassten Phasenwerts zu ermitteln.

Gemäß einer noch weiter bevorzugten Weiterbildung kann die Auswertevorrichtung dazu ausgebildet sein, sowohl den erfassten Intensitätswert als auch den erfassten Phasenwert unter Verwendung von wenigstens einem die Sensoranordnung charakterisierenden Systemparameter oder/und von wenigstens einem dem Erfassungsprozess entstammenden Prozessparameter in einen dimensionslosen erfassten Intensitätswert, bevorzugt in einen dimensionslosen normierten erfassten Intensitätswert, und in einen dimensionslosen erfassten Phasenwert, bevorzugt in einen dimensionslosen normierten erfassten Phasenwert, zu transformieren, wobei der vorbestimmte Kompensations-Wertezusammenhang ein vorbestimmter dimensionsloser Kompensations-Wertezusammenhang ist, nach dessen Maßgabe mit Eingangsgrößen auf Grundlage des dimensionslosen erfassten Intensitätswerts und des dimensionslosen erfassten Phasenwerts, der, vorzugsweise dimensionslose, besonders bevorzugt dimensionslose normierte, degradationskompensierte erfasste Wert ermittelt wird.

Entsprechend der obigen Beschreibung des Verfahrens kann der degradationskompensierte erfasste Wert ein dimensionsloser, bevorzugt ein dimensionsloser normierter, degradationskompensierter erfasster Wert sein und kann der vorbestimmte Kalibrations-Wertezusammenhang ein dimensionsloser, bevorzugt ein dimensionsloser normierter, vorbestimmter Kalibrations-Wertezusammenhang sein. Die Auswertevorrichtung ist dann bevorzugt dazu ausgebildet, den Ergebniswert nach Maßgabe des, vorzugsweise dimensionslosen, Kalibrations-Wertezusammenhangs mit einer Eingangsgröße auf Grundlage des dimensionslosen degradationskompensierten Werts zu ermitteln. Alternativ kann die Auswertevorrichtung dazu ausgebildet sein, einen dimensionslosen, bevorzugt dimensionslosen normierten degradationskompensierten Wert in einen dimensionsbehafteten degradationskompensierten Wert umzurechnen und auf diesen unter Anwendung des Kalibrations-Wertezusammenhangs den Ergebniswert zu berechnen.

Die oben genannte Aufgabe wird außerdem gelöst durch eine Messanordnung umfassend eine wie oben beschrieben ausgebildete Auswertevorrichtung sowie eine Sensoranordnung mit einem mit der Zeit degradierenden Luminophor, mit einer Anregungsstrahlungsquelle sowie mit wenigstens einem optischen Sensor, wobei das Luminophor als Reaktion auf eine Bestrahlung mit einer vorgegebenen modulierten Anregungsstrahlung in Abhängigkeit vom Ausmaß eines Kontakts des Luminophors mit einer eine Lumineszenz des Luminophors löschenden Quencher-Substanz gemäß einer sensoranordnungstypischen Antwortcharakteristik eine von dem wenigstens einen optischen Sensor erfasste Antwortstrahlung abstrahlt.

Die vorliegende Erfindung wird nachfolgend anhand der beiliegenden Zeichnungen näher erläutert. Es stellt dar:

Figur 1      eine Teil-Explosionsansicht eines Gehäuses einer nach dem Prinzip der Lumineszenzlöschung arbeitenden Sensoranordnung,

Figur 2      eine grobschematische Querschnittansicht durch eine Sensoranordnung der vorliegenden Erfindung,

Figur 3      einen Plot einer Intensität einer Antwortstrahlung der Sensoranordnung von Fig. 2 als Funktion der Phasenverschiebung bzw. des Phasenwinkels,

Figur 4      einen Plot der Intensität einer Antwortstrahlung als Funktion des Phasenwinkels für eine frisch kalibrierte Sensoranordnung und für eine seit ihrer Kalibration degradierte Sensoranordnung,

Figur 5      einen Plot einer normierten dimensionslosen Intensität einer Antwortstrahlung als Funktion eines normierten dimensionslosen Phasenwinkels für eine frisch kalibrierte Sensoranordnung mit Erfassungswertepaaren, die an unterschiedlich degradierten Sensoranordnungen ermittelt wurden,

Figur 6      den Plot von Fig. 5 mit den Erfassungswertepaaren zugeordneten hypothetischen Phasenwerten, die mit einer nicht-degradierten Sensoranordnung erhalten worden wären,

Figur 7      den Plot von Fig. 6 mit den Erfassungswertepaaren zusätzlich zugeordneten kompensierten Phasenwerten, die anhand einer Kompensations-Funktionsgleichung als einem Kompensations-Wertezusammenhang erhalten wurden, und

Figur 8      normierte dimensionslose Plots von einer frisch kalibrierten Sensoranordnung im fabrikneuen Zustand und von einer frisch kalibrierten Sensoranordnung mit degradiertem Luminophor.

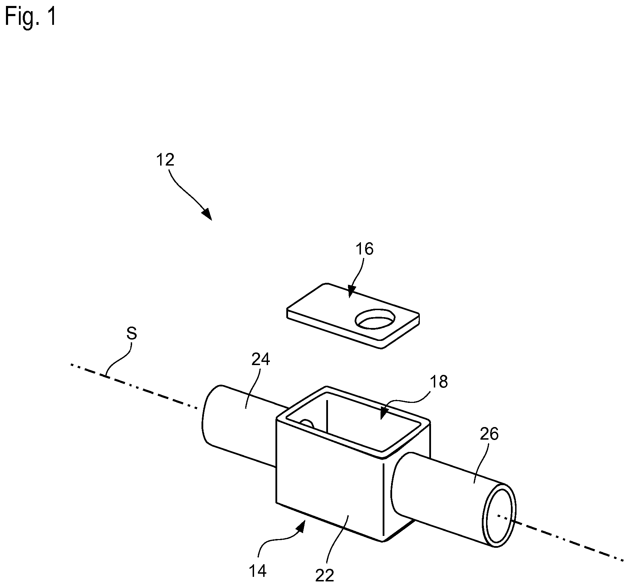

In Fig. 1 ist ein Gehäuse 12 einer nach dem Prinzip der Lumineszenzlöschung arbeitenden Sensoranordnung in einer Teil-Explosionsansicht dargestellt.

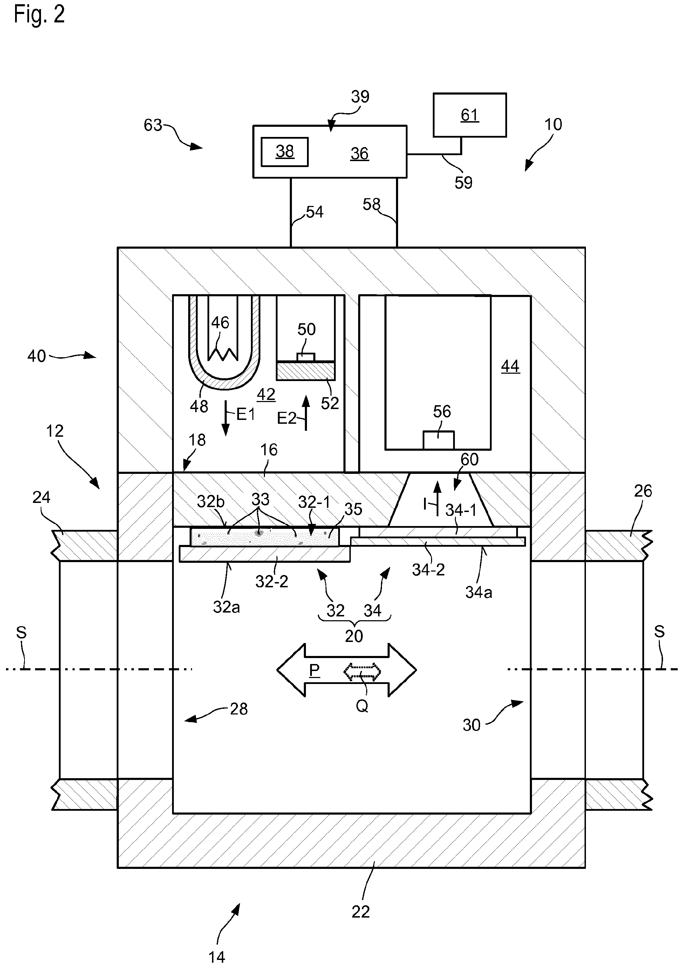

Das Gehäuse 12 umfasst ein Grundgehäuse 14 und ein Fensterbauteil 16 mit einer daran angeordneten, in Fig. 1 jedoch nicht zu erkennenden luminophorhaltigen Schichtbauteilanordnung 20 (s. Fig. 2). Mit dem Fensterbauteil 16 kann eine Öffnung 18 im Grundgehäuse 14 verschlossen werden.

Das Gehäuse 12 weist zu beiden Seiten des unter Beteiligung des Fensterbauteils 16 gebildeten parallelepipedförmigen Abschnitts 22 Anschlussstutzen 24 und 26 zum Anschluss von Fluidleitungsabschnitten daran auf.

Das Gehäuse 12 ist bidirektional längs der Strömungsachse S durchströmbar.

In Fig. 2 ist die allgemein mit 10 bezeichnete Sensoranordnung grobschematisch im Querschnitt dargestellt.

Das Gehäuse 12 ist zwischen seinen beiden Öffnungen 28 und 30 längs der Strömungsachse S bidirektional von Prüffluid P durchströmbar. Dabei strömt das Prüffluid P unter Kontaktierung von Fluidkontaktseiten 32a und 34a von Schichtkörpern 32 und 34 an diesen vorbei. Die Strömungsachse S liegt in der Zeichenebene von Fig. 2.

Die Sensoranordnung 10 ist im dargestellten Beispiel zur temperaturkompensierten luminophorbasierten Erfassung des Sauerstoff-Partialdrucks eines das Gehäuse 12 durchströmenden Prüffluids P, hier etwa Luft, ausgebildet. Sauerstoff bildet vorliegend also ein Beispiel für eine oben allgemein genannte Quencher-Substanz Q. Das Prüffluid kann ein Gas oder eine Flüssigkeit sein oder umfassen. Das Prüffluid kann eine Zweiphasen-Substanz mit sowohl einer Flüssigkeit als auch einem Gas sein. Das Prüffluid kann eine Suspension aus Flüssigkeit und Festkörperpartikeln, eine Emulsion und dergleichen Mischung sein. Beispielsweise kann die Sensoranordnung 10 zur Ermittlung des Sauerstoffgehalts in inspiratorischem oder/und exspiratorischem Atemgas ausgebildet sein, dass mittels einer Beatmungsvorrichtung einem Patienten zugeführt wird bzw. von diesem weggeführt wird.

**EP 3 794 334 B1**

Die Sensoranordnung 10 ist im vorliegenden Beispiel zur Temperaturkompensation ausgebildet. Dadurch können Erfassungssignale, die an einem Prüffluid P mit einer Temperatur erhalten wurden, die von der Kalibrationstemperatur eines zur Kalibration verwendeten Fluids mit bekannter Konzentration der Quencher-Substanz Q abweicht, in Erfassungssignale umgewandelt werden, die erhalten worden wären, wenn das Prüffluid P im Erfassungsvorgang die Kalibrationstemperatur aufgewiesen hätte. Die vorliegende Erfindung funktioniert jedoch auch an Sensoranordnungen, die nicht zur Temperaturkompensation ausgebildet sind.

Sowohl die Temperaturkompensation als auch die Umrechnung der unmittelbar erhaltenen Erfassungswerte in eine Sauerstoffkonzentration bzw. einen Sauerstoffgehalt des Prüffluids P erfolgt durch eine Steuereinrichtung 36 anhand einer in einem Datenspeicher 38 der Steuereinrichtung 36 hinterlegten Kalibrationsinformation. Die Kalibrationsinformation umfasst vorliegend auch, aber nicht nur, den oben genannten Kalibrations-Wertezusammenhang. Die Steuer- bzw. Recheneinrichtung 36 und der Datenspeicher 38 bilden gemeinsam eine Datenverarbeitungseinheit 39.

Eine Sensorbaugruppe 40, welche vorzugsweise lösbar am Gehäuse 12 angeordnet werden kann und hierzu beispielsweise den parallelepipedförmigen Abschnitt 22 von drei Seiten U-förmig umgibt, wobei die Basis des "U" dem Fensterbauteil 16 gegenüberliegt, umfasst im dargestellten Ausführungsbeispiel zwei Messkammern 42 und 44, welche baulich voneinander getrennt sind.

In der der Ermittlung der Sauerstoffkonzentration als einem gewünschten Ergebniswert dienenden Messkammer 42 ist eine Anregungsstrahlungsquelle 46, beispielsweise in Form einer LED, vorgesehen, welche eine elektromagnetische Anregungsstrahlung E1 einer ersten Wellenlänge ausstrahlt. Um das Wellenlängenband der von der Anregungsstrahlungsquelle 46 ausgehenden elektromagnetischen Anregungsstrahlung E1 möglichst eng zu halten und Störstrahlung zu vermeiden, kann die Anregungsstrahlungsquelle 46 von einem Filterkörper 48 umgeben sein, welcher die elektromagnetische Anregungsstrahlung E1 der genannten Wellenlänge mit einer möglichst kleinen Wellenlängentoleranz durchlässt.

Die Reaktionsschicht 32-1 des Schichtkörpers 32 enthält ein in einer Matrix 35 gehaltenes Luminophor 33, das durch die Anregungsstrahlung E1 zur Lumineszenz angeregt werden kann.

Weiter ist in der ersten Messkammer 42 ein Strahlungsdetektor 50 angeordnet, welcher eine von der Reaktionsschicht 32-1 nach deren Anregung durch die elektromagnetische Anregungsstrahlung E1 ausgehende elektromagnetische Antwortstrahlung E2 erfasst. Auch dem Strahlungsdetektor 50 kann ein Strahlungsfilter 52 vorgelagert sein, um nur die elektromagnetische Antwortstrahlung E2 mit ihrer von der Wellenlänge der Anregungsstrahlung E1 verschiedenen zweiten Wellenlänge durchzulassen. Durch die Filteranordnungen 48 und 52 kann sichergestellt werden, dass keine Strahlung direkt von der Anregungsstrahlungsquelle 46 zum Strahlungsdetektor 50 gelangt und das dort erfasste Signal "verrauscht".

Das vom Strahlungsdetektor 50 aufgrund seiner Erfassung der Antwortstrahlung E2 ausgegebene Signal wird über die in Fig. 1 gezeigte Datenleitung 54 an die Steuereinrichtung 36 übertragen. Es repräsentiert in an sich bekannter Weise den SauerstoffPartialdruck und damit den Ergebniswert für das das Gehäuse 12 durchströmende Prüffluid P.

In der zweiten Messkammer 44, welche optional vorhanden ist und der Temperaturkompensation des Erfassungssignals des Strahlungsdetektors 50 dient, ist ein Infrarot-Detektor 56 angeordnet, welcher von der Erfassungsschicht 34-1 abgestrahlte Infrarot-Strahlung I erfasst. Das vom Infrarot-Detektor 56 aufgrund seiner Erfassung der Infrarot-Strahlung I ausgegebene Signal wird über die Datenleitung 58 an die Steuereinrichtung 36 übertragen. Dieses Signal ist indikativ für eine Temperatur der Erfassungsschicht 34-1 und wegen der hohen Wärmeleitfähigkeit des Schichtkörpers 34 auch indikativ für die Temperatur der mit derselben Prüffluid-Strömung thermisch wechselwirkenden luminophorhaltigen Reaktionsschicht 32-1.

Aufgrund der im Datenspeicher 38 der Steuereinrichtung 36 hinterlegten Kalibrationsinformation, welche vor dem produktiven Einsatz der Sensoranordnung 10 in einem gesonderten Kalibrationsvorgang ermittelt wurde, kann die Steuereinrichtung 36 für jeden Erfassungszeitpunkt eines Signals des Strahlungsdetektors 50 die Temperatur der Reaktionsschicht 32-1 aus dem Erfassungssignal des Infrarot-Detektors 56 ermitteln und so das Erfassungssignal des Strahlungsdetektors 50 bezüglich der Temperatur des abstrahlenden Reaktionsschichtkörpers 32 bzw. der Reaktionsschicht 32-1 desselben kompensieren. Das Ergebnis ist eine hochgenaue Bestimmung des Sauerstoff-Partialdrucks in dem das Gehäuse 12 durchströmenden Prüffluid P.

Die hochgenaue Temperaturkompensation wird dabei mit äußerst einfachen Mitteln erreicht, wie beispielsweise einer Metallfolie als Trägerschicht 34-2 und der darauf aufgetragenen Erfassungsschicht 34-1. Die Erfassungsschicht 34-1 umfasst einen oder ist bevorzugt ein karbon-haltiger Lack mit Karbon als schwarzen Farbpigmenten. Der karbonhaltige Lack weist daher einen sehr hohen Emissionsgrad von mehr als 0,9 auf. Die Verwendung der Metallfolie als Trägerschicht 34-1, aus Gründen möglichst guter Wärmeleitung beispielhaft gebildet aus einer Aluminiumfolie, mit bevorzugt nicht mehr als 12 μm Dicke, gestattet, im Fensterbauteil 16 bzw. allgemein im Gehäuse 12 ein das Fensterbauteil 16 bzw. das Gehäuse 12 durchsetzendes Loch 60 auszubilden, das vom Schichtkörper 34 vollständig bedeckt ist. Dadurch erreicht die von der Erfassungsschicht 34-1 als Infrarot-Strahlung I abgestrahlte Temperaturinformation mit geringstmöglicher Verfälschung den Infrarot-Detektor 56.

Bei einer nicht zur Temperaturkompensation ausgebildeten Sensoranordnung 10 entfallen die Messkammer 44 mit dem Infrarotdetektor 56, der Erfassungsschichtkörper 34 und das Loch 60.

Soweit zur mittelbaren Ermittlung der Temperatur der Reaktionsschicht 32-1 und deren Berücksichtigung bei der Ermittlung des Ergebniswerts aus den vom Strahlungsdetektor 50 erhaltenen Erfassungssignalen. Nun zurück zur Auswertung der Erfassungssignale des Strahlungsdetektors 50.

Die optische, luminophorbasierte Erfassung einer Sauerstoffkonzentration, etwa in Form des Sauerstoff-Partialdrucks, in einem Prüffluid P ist an sich bekannt. Sie erfolgt im vorliegenden Ausführungsbeispiel unter Beteiligung des Reaktionsschichtkörpers 32. Vorliegend ist der Reaktionsschichtkörper 32 zweilagig. Tatsächlich kann der Reaktionsschichtkörper 32 nur eine oder auch mehr als zwei Schichten aufweisen. Im dargestellten Beispiel - zu erkennen in der Querschnittsansicht von Fig. 2 - weist der Reaktionsschichtkörper 32 eine Trägerlage 32-2 und die darauf aufgetragene luminophorhaltige Reaktionsschicht 32-1 auf.

Die Verhältnisse von Länge und Breite des Reaktionsschichtkörpers 32 zu seiner Dicke sind in den Figuren nicht maßstäblich. Der dargestellte Reaktionsschichtkörper 32 kann eine Kantenlänge von etwa 7 bis 10 mm aufweisen, wobei seine Dicke über beide Schichten 32-1 und 32-2 gemessen etwa 300 μm betragen kann.

Die Trägerlage 32-2 kann dabei aus einem für Sauerstoffmoleküle ausreichend porösen Werkstoff gebildet sein, wie beispielsweise Polyvinylidenfluorid. Die Trägerlage 32-2 kann aus einer entsprechenden Folie ausgeschnitten sein und eine Dicke von zwischen 100 bis 150 μm aufweisen. Unter Umständen kann die Dicke der Trägerlage 32-2 auch geringer sein.

Die luminophorhaltige Reaktionsschicht 32-1 kann ebenfalls Polyvinylidenfluorid als Matrixmaterial enthalten, in welches Luminophore eingebettet sind.

Die luminophorhaltige Reaktionsschicht 32-1 kann etwas kleiner ausgebildet sein als die sie tragende Trägerlage 32-2, um die adhäsive Anbringung des Reaktionsschichtkörpers 32 mit der Erfassungsseite an dem Fensterbauteil 16 oder allgemein an dem Gehäuse 12 zu erleichtern, ohne hierdurch die Erfassungsseite 32b der luminophorhaltigen Reaktionsschicht 32-1 mit Klebstoff belegen zu müssen.

Auch die Darstellung des Temperatur-Erfassungsschichtkörpers 34 ist hinsichtlich seiner Abmessungen nicht maßstäblich. Er weist im dargestellten Beispiel eine Kantenlänge in dem identischen Bereich auf wie der Reaktionsschichtkörper 32, ist jedoch aufgrund seines vom Reaktionsschichtkörper 32 abweichenden Aufbaus in der Regel dünner als dieser.

Die den Detektoren 50 und 56 zugewandten Erfassungsseiten (siehe Erfassungsseite 32b) der beiden Schichtkörper 32 und 34 sind dabei vorteilhafterweise nach außen, also vom Prüffluid P weg gerichtet, während die Fluidkontaktseite 32a bzw. 34a der beiden Schichtkörper möglichst großflächig mit dem Fluid in Kontakt gelangt.

Um sicherzustellen, dass die Reaktionsschicht 32-1 nur von im Prüffluid P gelöstem Sauerstoff erreicht wird, ist der Reaktionsschichtkörper auf seiner Erfassungsseite 32b von dem Fensterbauteil 16 abgedeckt. Das Fensterbauteil 16 kann aus einem transparenten Polyamid oder auch aus einem anderen für die Anregungs- und für die Antwortstrahlung durchlässigen Kunststoff gebildet sein. Beispielsweise kann das Fensterbauteil 16 aus amorphem Polyamid gebildet sein, wie er unter dem Namen "Grilamid TR ®" von der Firma EMS-Chemie AG in Domat (CH) angeboten wird.

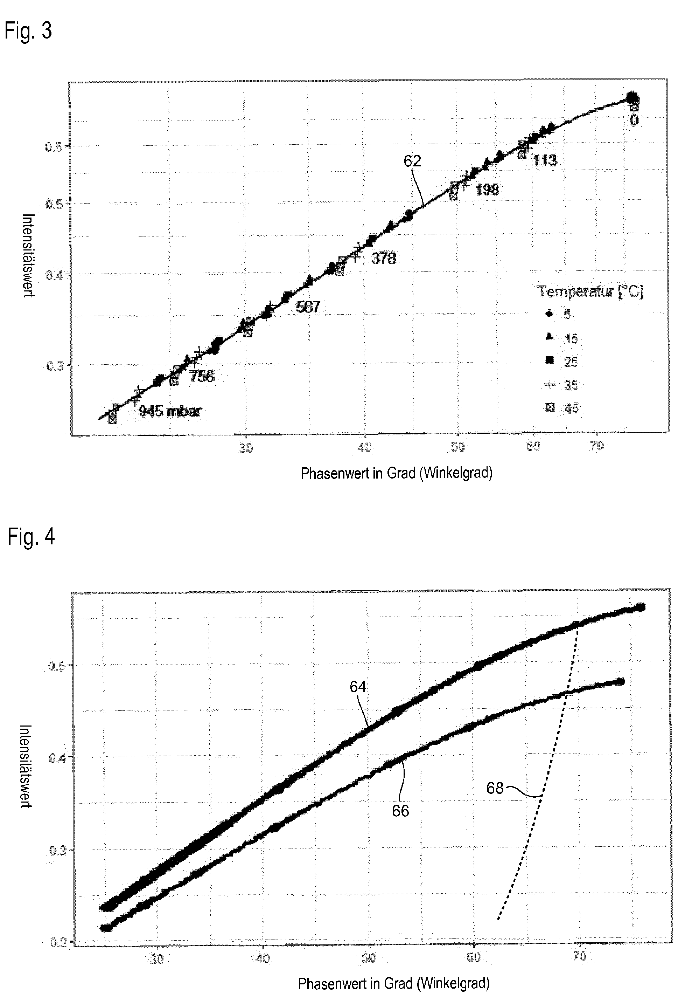

In Figur 3 ist ein Graph eines Funktionszusammenhangs zwischen einem auf der Abszisse aufgetragenen Phasenwert in Grad und einem längs der Ordinate aufgetragenen Intensitätswert dargestellt. Der Graph ist mit Bezugszeichen 62 bezeichnet.

Beim Betrieb der Sensoranordnung 10 strahlt die Anregungsquelle 46 die Anregungsstrahlung E1 mit einer vorbestimmten Intensitätsmodulation aus.

Die von der luminophorhaltigen Reaktionsschicht 32-1 in Antwort auf die modulierte Anregungsstrahlung E1 ausgestrahlte Antwortstrahlung E2 weist daher ebenfalls eine modulierte Intensität auf. Somit sind zwei Erfassungswerte der Sensoranordnung 10 aus der Antwortstrahlung E2 erhältlich: ein die Intensität der Antwortstrahlung E2 repräsentierender Intensitätswert und ein den Phasenversatz zwischen den beiden modulierten Strahlungen angebender Phasenwert. Der Intensitätswert kann beispielsweise als Verhältnis der Intensität der Antwortstrahlung E2 zur Intensität der Anregungsstrahlung E1 ausgegeben werden.

Da, wie oben beschrieben, eine Wechselwirkung mit einer Quencher-Substanz Q, vorliegend beispielsweise Sauerstoff, im Prüffluid P abhängig von der Menge an im Prüffluid P vorhandenen Quencher-Substanz Q die Lumineszenz des Luminophors in der Reaktionsschicht 32-1 ablöscht und somit sowohl den Intensitätswert wie auch den Phasenwert der Antwortstrahlung E2 beeinflusst, kann aus jedem einzelnen der beiden Erfassungswerte: Intensitätswert und Phasenwert, mittels eines Kalibrations-Wertezusammenhangs auf die Konzentration der Quencher-Substanz Q im Prüffluid P geschlossen werden.

Beispielsweise kann für einen Phasenwert $\Phi$ ein Kalibrations-Wertezusammenhang durch die nachfolgend angegebene kalibrierte Stern-Volmer-Gleichung angegeben sein:

$$\frac{tan(\phi)}{tan(\phi_0(T))} = \frac{m_2}{1+K_{sv}(T)\,p_{O_2}} + \frac{1-m_2}{1+m_1 K_{sv}(T)\,p_{O_2}} \qquad \text{(Gl. 1)}$$

mit dem erfassten Phasenwert $\Phi$ als beispielhaftem Erfassungswert, $\Phi_0(T)$ als dem vorbekannten oder experimentell ermittelten temperaturabhängigen Phasenwert bei völliger Abwesenheit der Quencher-Substanz Q, $K_{sv}(T)$ als vorbekanntem oder experimentell ermitteltem temperaturabhängigem Stern-Volmer-Koeffizienten, mit $p_{O_2}$ als dem Sauerstoffpartialdruck als beispielhaftem Ergebniswert, mit $m_1$ und $m_2$ als beim Kalibrationsvorgang ermittelten Kalibrationskonstanten.

Gleichung 1 als beispielhafter Kalibrations-Wertezusammenhang ist bei erfasstem Phasenwert $\Phi$ numerisch oder analytisch für den Sauerstoffpartialdruck $p_{O_2}$ als dem Ergebniswert lösbar. Somit kann unmittelbar der Sauerstoffpartialdruck aus dem erfassten Phasenwert ermittelt werden.

Zur Ermittlung des Sauerstoffpartialdrucks als beispielhafter Ergebniswert, welcher eine Konzentration der Quencher-Substanz Q im Prüffluid P repräsentiert, reicht somit einer der beiden möglichen Erfassungswerte aus.

Das in der Reaktionsschicht 32-1 der Sensoranordnung 10 von Figur 2 enthaltene Luminophor gibt zwar stets in Antwort auf eine Anregungsstrahlung E1 eine Antwortstrahlung E2 aus. Jedoch ändert sich aufgrund von Alterung von Luminophors, beispielsweise durch Ausbleichen desselben, die materialimmanente Antwortcharakteristik, welche das Lumineszenzverhalten des Luminophors in Antwort auf die Anregungsstrahlung E1 bestimmt.

Für ein und dieselbe Anregungsstrahlung E1 und für ein und dieselbe Konzentration der Quencher-Substanz Q wird mit ein und derselben Sensoranordnung 10 abhängig vom Degradationszustand der Sensoranordnung 10 jeweils eine andere Antwortstrahlung E2 und werden somit unterschiedliche Erfassungswerte: Intensitätswert und Phasenwert, erhalten.

Die Degradation des Luminophors bewirkt, dass sich die möglichen Erfassungswerte: Intensitätswert und Phasenwert, ausgehend von einem Kalibrationszustand, dem eine zum Zeitpunkt der Kalibration vorherrschende Bezugs-Antwortcharakteristik des Luminophors zu Grunde liegt, hin zu niedrigeren Werten sowohl der Intensität als auch des Phasenwinkels (Phasenwert) ändern.

Ein mit der Sensoranordnung 10 arbeitender Laborant, der beispielsweise aufgrund eines Erfassungsvorgangs für ein Prüffluid nur einen Phasenwert als Erfassungswert erhält, kann nur in zeitlich ausreichender Nähe zum letzten Kalibrationsvorgang, bei welchem der immer noch verwendete Kalibrations-Wertezusammenhang ermittelt wurde, sicher sein, dass er aus dem erhaltenen Erfassungswert mittels des Kalibrations-Wertezusammenhangs auf den korrekten Ergebniswert schließen kann.

Besteht dagegen das Risiko, dass das Luminophor seit der letzten Kalibration alterungsbedingt degradiert ist, ist ohne weitere Maßnahmen nicht ermittelbar, ob ein niedriger erhaltener Erfassungswert durch eine tatsächlich höhere Konzentration der Quencher-Substanz Q im Prüffluid P bewirkt ist oder ob eine altersbedingte Degradation des Luminophors für den niedrigen Wert verantwortlich ist. Auf Grundlage eines degradationsbedingt zu niedrigen Erfassungswerts wird bei vorgegebenem Kalibrations-Wertezusammenhang ein zu hoher Ergebniswert ermittelt.

Wie die Erfinder der vorliegenden Anmeldung jedoch herausgefunden haben, stehen die für eine vorbestimmte Konzentration der Quencher-Substanz Q im Prüffluid P erhaltenen Erfassungswerte: Intensitätswert und Phasenwert, in einem eindeutigen Funktionszusammenhang. Der Graph 62 zeigt diesen Funktionszusammenhang graphisch an. Überraschend bleibt dieser Funktionszusammenhang auch dann bestehen, wenn sich die Temperatur des Prüffluids P ändert. Für den Graphen 62 wurden Erfassungsvorgänge mit unterschiedlichen Partialdrücken der Quencher-Substanz Q im Prüffluid P bei Temperaturen von 5 bis 45 °C in Sprüngen von 10 K durchgeführt. Dabei wurde jeweils ein Prüffluid P mit bekannter Konzentration der Quencher-Substanz Q verwendet, nämlich mit einem Partialdruck, gemessen in Millibar (hPa), mit den Beträgen 0, 113, 189, 378, 567, 756 und 945. Die durch die beispielhaften Messungen erhaltenen Erfassungswertepaare, die alle für die gleiche Antwortcharakteristik des Luminophors bzw. der luminophorhaltigen Reaktionsschicht 32-1 erhalten wurden, können beispielsweise mit der Methode der kleinsten Fehlerquadrate in eine Gleichung geeigneter Struktur analytisch dargestellt werden.

Figur 4 zeigt mit Bezugszeichen 64 qualitativ einen Zusammenhang von Intensitätswert und Phasenwert für eine fabrikneue Sensoranordnung 10. Die Darstellung entspricht qualitativ dem Graphen 62 von Figur 3. Im Vergleich hierzu zeigt der Graph 66 in Figur 4 einen Phasenwert-Intensitätswert-Zusammenhang derselben Sensoranordnung, jedoch mit gealtertem Luminophor. Ein Vergleich der in Figur 4 jeweiligen rechten Enden der Graphen 64 und 66, welche die Erfassungswerte: Intensitätswert und Phasenwert, für ein Prüffluid mit völliger Abwesenheit der Quencher-Substanz Q zeigen, illustriert, wie mit der altersbedingten Degradation des Luminophors in der Sensoranordnung 10 bei gleicher Anregungsstrahlung E1 sowohl der Intensitätswert als auch der Phasenwert der durch die Anregungsstrahlung E1 provozierten Antwortstrahlung E2 abnimmt.

Aufgrund des eindeutigen Zusammenhangs von Intensitätswert und Phasenwert kann jedoch sofort ermittelt werden, ob ein Erfassungswert mit einem degradierten Luminophor oder mit einem nicht degradierten Luminophor erhalten wurde. Dabei bezieht sich die Degradation jeweils auf den letzten Kalibrationszustand, für welchen die frisch kalibrierte Sensoranordnung 10 jeweils tadellos funktioniert. Die Graphen 64 und 66 in Figur 4 sind daher repräsentativ für die jeweiligen Antwortcharakteristika des Luminophors in der Sensoranordnung 10 einmal im Kalibrationszustand (siehe 64) und einmal in einem davon abweichenden Degradationszustand (siehe 66).

Wird beispielsweise die fabrikneue Sensoranordnung 10 kalibriert, repräsentiert der Phasenwert-Intensitätswert-Zusammenhang 64 die Bezugs-Antwortcharakteristik. Die Sensoranordnung 10 kann so lange für Erfassungen von Konzentrationen von Quencher-Substanz Q im Prüffluid P herangezogen werden, wie sich die Antwortcharakteristik des Luminophors gegenüber der Bezugs-Antwort-Charakteristik 64 nicht ändert.

Hat sich die Antwortcharakteristik jedoch mit fortschreitender Degradation beispielsweise hin zum Graphen 66 entwickelt, liefert die Sensoranordnung 10 für die jeweilige Konzentration an Quencher-Substanz Q im Prüffluid P zu niedrige Erfassungswerte und damit letztendlich falsche Ergebniswerte. Wird jedoch die Sensoranordnung 10 in diesem Degradationszustand erneut kalibriert, wird der durch den Graphen 66 repräsentierte Phasenwert-Intensitätswert-Zusammenhang zur Bezugs-Antwortcharakteristik und die Sensoranordnung 10 liefert wieder korrekte Ergebniswerte. Jedoch wird die Sensoranordnung 10 weiter degradieren, sodass einige Zeit nach der letzten Kalibration wieder falsche Erfassungswerte erhalten werden.

Durch Betrachtung nicht nur eines Erfassungswertes, sondern beider Erfassungswerte: Intensitätswert und Phasenwert, kann jedoch sofort ermittelt werden, ob der Betriebszustand der Sensoranordnung dem Kalibrationszustand ausreichend entspricht, oder ob er sich über eine Toleranzschwelle hinaus von diesem so weit entfernt hat, dass eine erneute Kalibration notwendig ist. Kalibrationsvorgänge sind unproduktiv und daher teuer. Ziel ist es daher, die Häufigkeit einer Kalibration der Sensoranordnung 10 so weit es geht zu reduzieren.

Eine strichliniert in Figur 4 gezeichnete Spur 68 gibt qualitativ an, wie sich für ein Prüffluid P mit einer vorgegebenen konstanten Konzentration der Quencher-Substanz Q der Phasenwert und der Intensitätswert mit fortschreitender Degradation des Luminophors in der Sensoranordnung 10 verändern. Durch wiederholte Kalibration kann in jedem beliebigen Degradationszustand der jeweils erhaltene Erfassungswert auf der Spur 68 mit dem korrekten Ergebniswert, nämlich der vorbekannten konstanten Konzentration der Quencher-Substanz Q verknüpft werden.

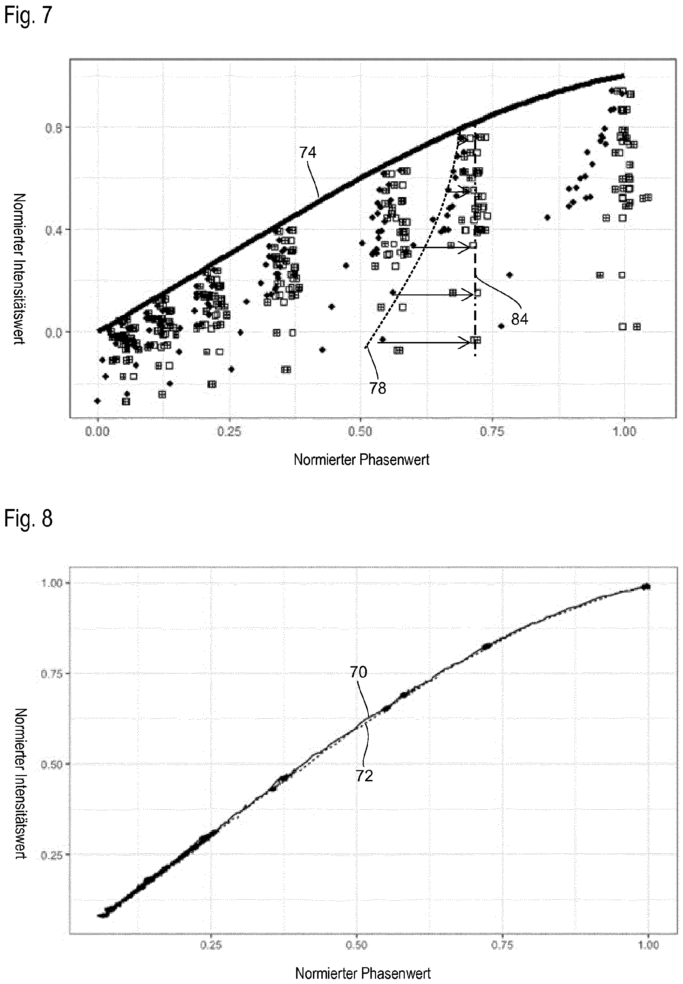

Figur 8 zeigt dabei einen erstaunlichen Zusammenhang. Normiert man die beiden Graphen 64 und 66 unterschiedlicher Degradationszustände derart, dass sie Erfassungswerte betragsmäßig jeweils nur zwischen den Extremwerten 0 und 1 aufweisen können, erhält man die normierten dimensionslosen Kurven 70 und 72 für die Graphen 64 bzw. 66 von Figur 4. Die beiden Kurven sind in der normierten dimensionslosen Notation identisch.

Die Normierung kann beispielsweise für den erfassten Phasenwert $\Phi$ und den erfassten Intensitätswert I gemäß den nachfolgend dargestellten Gleichungen 2 und 3 erfolgen:

$$\phi_N = \frac{\phi - \phi_{min}}{\phi_{max} - \phi_{min}} \tag{Gl. 2}$$

$$I_N = \frac{I - I_{min}}{I_{max} - I_{min}} \tag{Gl. 3}$$

wobei $\Phi_N$ der normierte dimensionslose Phasenwert ist und wobei $I_N$ der normierte dimensionslose Intensitätswert ist. Mit dem Index *max* ist der jeweils betragsmäßig größte mögliche Erfassungswert bezeichnet, etwa bei völliger Abwesenheit der Quencher-Substanz Q, und mit dem Index *min* der jeweils betragsmäßig kleinste mögliche Erfassungswert, etwa bei Verwendung reiner Quencher-Substanz Q als Prüffluid P.

Die für die Normierung benötigten Extremwerte $\Phi_{max}$, $\Phi_{min}$, $I_{max}$ und $I_{min}$ können entweder experimentell bestimmt werden, oder analytisch durch nachfolgend beispielhaft gezeigte Näherungsrechnung:
Beispielsweise kann der normierte Phasenwert-Intensitätswert-Zusammenhang von Figur 8 für eine neue Sensoranordnung 10 dieses Sensoranordnungstyps durch folgendes Polynom beschrieben werden:

$$_aI_N(\phi_N) = \alpha\,\phi_N + \beta\,\phi_N^2 + (1 - \alpha - \beta)\,\phi_N^3 \tag{Gl. 4}$$

mit den Randbedingungen $_aI_N(0) = 0$ und $_aI_N(1) = 1$.

Mit üblichen Fitting-Methoden erhält man für die Koeffizienten $\alpha$ und $\beta$ die Werte $\alpha$ = 1,17954 und $\beta$ = 0,26311. Gleichung 4 beschreibt somit eine Antwortcharakteristik der Sensoranordnung 10 bzw. des Sensoranordnungstyps der Sensoranordnung 10. Sie ist bevorzugt im Datenspeicher 38 gespeichert.

Die Extremwerte $\Phi_{max}$ und $\Phi_{min}$ kann man unmittelbar bei oder nach der Kalibration aus Gleichung 1 mit $p_{o2}$ = 0 (für $\Phi_{max}$) und mit $p_{o2} = p_{o2,max}$ (für $\Phi_{min}$) direkt errechnen.

Für die zur Kalibration verwendeten dimensionsbehafteten Wertepaare ($\Phi_i$, $I_i$) können mit dann mit bekannten $\Phi_{max}$, $\Phi_{min}$, aus Gleichung 2 die normierten dimensionslosen Phasenwerte $\Phi_{N,i}$ errechnet werden. Da aus den Gleichungen 3 und 4 gelten muss

$$_aI_{N,i} = \frac{_aI_N(\Phi_{N,i}) - I_{min}}{I_{max} - I_{min}} \tag{Gl. 5}$$

können aus Gleichung 5 die Extremwerte $I_{max}$ und $I_{min}$ ermittelt werden durch gegebenenfalls numerische Lösung des folgenden Gleichungssystems:

$$I_{max} = \frac{I_2\,(1+\beta(\phi_{N,1}-1)\phi_{N,1}^2-\phi_{N,1}^3+\alpha\,\phi_{N,1}(\phi_{N,1}^2-1))+I_1(\phi_{N,2}^3+\alpha\,\phi_{N,2}\,(1-\phi_{N,2}^2)-1-\beta(\phi_{N,2}-1)\phi_{N,2}^2)}{-\phi_{N,1}^3+\phi_{N,2}^3+\alpha(-\phi_{N,1}+\phi_{N,1}^3+\phi_{N,2}-\phi_{N,2}^3)+\beta(-\phi_{N,1}^2+\phi_{N,1}^3+\phi_{N,2}^2-\phi_{N,2}^3)}\ \text{(Gl. 6)}$$

$$I_{min} = \frac{I_2\,\phi_{N,1}\big((\beta(\phi_{N,1}-1)-\phi_{N,1})\phi_{N,1}+\alpha(\phi_{N,1}^2-1)\big)+I_1\phi_{N,2}(\alpha(1-\phi_{N,2}^2)+\phi_{N,2}(\beta+(1-\beta)\,\phi_{N,2}))}{-\phi_{N,1}^3+\phi_{N,2}^3+\alpha(-\phi_{N,1}+\phi_{N,1}^3+\phi_{N,2}-\phi_{N,2}^3)+\beta(-\phi_{N,1}^2+\phi_{N,1}^3+\phi_{N,2}^2-\phi_{N,2}^3)},\ \text{(Gl. 7)}$$

Die Bestimmung der Extremwerte muss jedoch nur bei der Kalibration ausgeführt werden. Die Verwendung von lediglich zwei Wertepaaren mit $j$ = 1, 2 reicht dabei aus.

Durch die so betragsmäßig bestimmten Extremwerte $\Phi_{max}$, $\Phi_{min}$, $I_{max}$ und $I_{min}$ können für jeden Erfassungs-vorgang der Sensoranordnung 10 oder einer Sensoranordnung des gleichen Sensoranordnungstyps aus den Gleichungen 2 und 3 unmittelbar und sofort aus den erhaltenen dimensionsbehafteten Erfassungswerten $\Phi_j$, $I_j$ die zugehörigen dimensionslosen normierten Erfassungswerte $\Phi_{N,j}$, $I_{N,j}$ ohne großen Aufwand ermittelt werden.

Durch Vergleich der so ermittelten normierten Erfassungswerte $\Phi_{N,j}$, $I_{N,j}$ mit den aus Gleichung 4 erhaltenen Werten $_aI_{N,j}(\phi_{N,j})$ kann sofort bestimmt werden, ob das Luminophor der Sensoranordnung 10 degradiert ist oder nicht. Ergibt eine solche Bestimmung, dass das Luminophor degradiert ist, gibt die Steuereinrichtung 36 über die Datenleitung 59 einen optischen oder/und akustischen Warnhinweis an eine Ausgabeeinrichtung 61 aus, wo sie vom Bedienpersonal der Sensoranordnung 10 wahrgenommen werden kann.

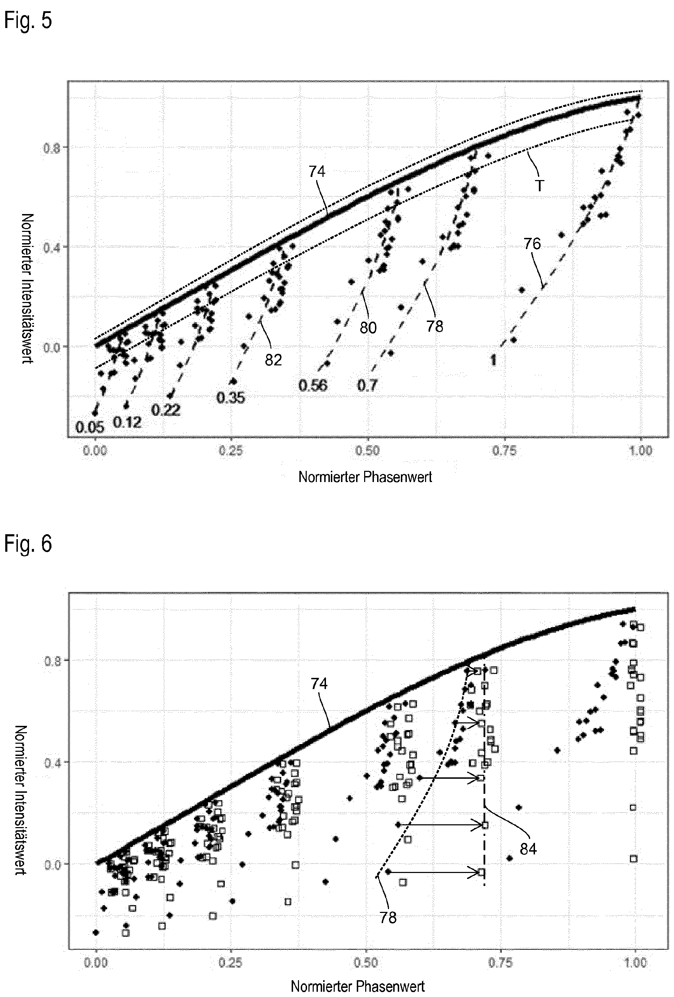

Die Antwortcharakteristik der Gleichung 4 ist in Fig. 5 als Bezugs-Antwortcharakteristik als Graph 74 gezeigt. Solange die Antwortcharakteristik der Sensoranordnung 10 (oder einer Sensoranordnung desselben Typs) mit der Bezugs-Antwortcharakteristik zur Zeit der Kalibration übereinstimmt, liefert die Sensoranordnung 10 Wertepaare $\Phi_{N,j}$, $I_{N,j}$ ,die auf dem Graphen 74 liegen. Ein auf dem Graphen 74 basierender Kalibrations-Wertezusammenhang, welcher Erfassungswerte der Sensoranordnung 10 mit zugehörigen Ergebniswerten für die Konzentration bzw. den Partialdruck der Quencher-Substanz Q im Prüffluid P verknüpft, liefert korrekte Ergebniswerte.

Mit zunehmender Degradation weichen die für ein Prüffluid erhaltenen Erfassungswertepaare $\Phi_{N,j}$, $I_{N,j}$ jedoch von dem Graphen 74 ab. Die Abweichung erfolgt mit zunehmender Degradation entlang der in Fig. 5 gezeigten Spuren, von welchen der Übersichtlichkeit halber lediglich die vier ganz rechts gelegenen Spuren mit den Bezugszeichen 76 bis 82 bezeichnet sind. Liegen die erhaltenen Erfassungswertepaare außerhalb des Toleranzbandes T, schließt die Steuereinrichtung 36 auf eine nicht mehr tolerierbare Degradation des Luminophors und gibt den Warnhinweis aus.

Jede dieser Spuren zeigt den Verlauf von Erfassungswertepaaren, die mit zunehmender Degradation für ein Prüffluid P mit konstanter Konzentration an Quencher-Substanz Q erhalten werden. Die in Fig. 5 gezeigten Spuren sind Iso-Konzentrationslinien, die die von der Sensoranordnung 10 ausgegebenen Erfassungswertepaare für jeweils identische Prüffluide P mit jeweils konstanter Konzentration der Quencher-Substanz Q in unterschiedlichen Degradationszuständen anzeigen. So gibt die Spur 80 in Fig. 5 beispielsweise den Verlauf jener Erfassungswertepaare an, die ausgehend von der Bezugs-Antwortcharakteristik 74 für dasjenige Prüffluid erhalten werden, welches auf Grundlage der Bezugs-Antwortcharakteristik einen normierten dimensionslosen Phasenwert von 0,7 liefert. Mit fortschreitender Degradation werden auch im normierten dimensionslosen Werteraum die normierten dimensionslosen Phasenwerte zunehmend kleiner als bei Verwendung einer seit der letzten Kalibration noch nicht degradierten Sensoranordnung.

Iso-Konzentrationslinien könnten auch in der dimensionsbehafteten Darstellung der Fig. 4 eingetragen werden, wie die Spur 68 dort zeigt, die ebenfalls eine Iso-Konzentrationslinie ist. Der Begriff "Iso-Konzentrationslinie" ist dabei gleichbedeutend mit dem Begriff "Iso-Partialdrucklinie".

Erfasst man bei der Erstellung des Plots von Fig. 5 mit einer oder mehreren weiteren, frisch kalibrierten Sensoranordnungen für jeden Erfassungspunkt die tatsächliche Konzentration (Partialdruck) der Quencher-Substanz Q im Prüffluid P, so kann mittels der obigen Gleichung 1 bei bekanntem Sauerstoffpartialdruck $p_{o_2}$ jener hypothetische Phasenwert $\Phi_{hyp}$ ermittelt werden, der anstelle des tatsächlich mit der Sensoranordnung 10 in unterschiedlichen Degradationszuständen ermittelten Phasenwerts ermittelt worden wäre, befände die Sensoranordnung 10 während des jeweiligen Erfassungsvorgangs im Kalibrationszustand.

Die Darstellung des Plots von Fig. 5 mit einem einem jeden tatsächlichen Erfassungswertepaar derart zugeordnetem hypothetischen Erfassungswert in Gestalt des hypothetischen Phasenwerts $\Phi_{hyp}$ ist in Fig. 6 dargestellt. Tatsächlich erfasste Erfassungswertepaare (so auch genannte "tatsächliche Erfassungswertepaare") sind in den Fig. 5 bis 7 als massive Raute dargestellt. Der einem tatsächlichen Erfassungswertepaar zugeordnete hypothetische Phasen-wert $\Phi_{hyp}$ ist in den Fig. 5 bis 7 als ungefülltes Quadrat dargestellt.

Für fünf tatsächliche Erfassungswertepaare ist in Fig. 6 mit dünnen Pfeilen jeweils die Abweichung ihres tatsächlich erfassten Phasenwerts von dem ausgehend von der bekannten Konzentration mit Gleichung 1 bestimmten

hypothetischen Phasenwert $\Phi_{hyp}$ dargestellt. Eine hypothetische Spur 84 zeigt den Verlauf der hypothetischen Phasenwerte $\Phi_{hyp}$ für die der Spur 78 zugeordneten tatsächlichen Erfassungswerte an.

Eine Abbildungsvorschrift, welche tatsächlich erfassten Erfassungswertepaare zumindest bezüglich eines Erfassungswertes, im dargestellten Beispiel: des Phasenwerts, auf den zugeordneten hypothetischen Erfassungswert abbildet, stellt somit eine Degradationskompensation bereit, die den für die Ermittlung des Ergebniswerts benötigten Erfassungswert von einem tatsächlichen Erfassungswert auf einen hypothetischen Erfassungswert einer nicht-degradierten Sensoranordnung überführt.

Eine mögliche solche Abbildungsvorschrift ist beispielhaft die nachfolgende Gleichung 8:

$$\psi_N(\phi_N, I_N) = a\,\phi_N + b\,\phi_N^2 + c\,I_N + d\,I_N\,\phi_N + e\,I_N^2 \qquad \text{(Gl. 8)}$$

Diese Gleichung 8 bildet normierte dimensionslose Erfassungswertepaare ($\Phi_N$, $I_N$) als normierte dimensionslose degradationskompensierte Erfassungswerte, hier degradationskompensierte Phasenwerte $\psi_N$, ab. Gleichung 8 nutzt folglich sowohl die erfasste Intensität als auch den erfassten Phasenwert eines Erfassungsvorgangs, um den Phasenwert hinsichtlich einer möglicherweise eingetretenen Degradation des Luminophors zu kompensieren und folglich einen Modell-Phasenwert auszugeben, welcher von der Sensoranordnung 10 erhalten worden wäre, wenn sich diese während des Erfassungsvorgangs im Kalibrationszustand befunden hätte. Experimentell konnten für den Sensoranordnungstyp der Sensoranordnung 10 die Parameterwerte $a$ = 1,20, $b$ = 0,2, $c$ = -0,14, $d$ = -0,5 und $e$ = 0,25 ermittelt werden.

Für die Anwendung der Abbildungsvorschrift der Gleichung 8 ist es dabei in besonders vorteilhafterweise unerheblich, ob das Luminophor der Sensoranordnung 10 tatsächlich degradiert ist oder nicht. Sie bildet Wertepaare, die der Gleichung 4 genügen, mit ausreichender Genauigkeit auf sich selbst ab.

Gleichung 8 ist daher ein Beispiel für einen Kompensations-Wertezusammenhang, wie er in der Beschreibungseinleitung genannt ist. Dieser ist im Datenspeicher 38 gespeichert.

Figur 7 zeigt für tatsächlich erfasste Erfassungswertepaare zusätzlich die jeweils zugeordneten degradationskompensierten normierten dimensionslosen Phasenwerte mit einem Fenstersymbol.

Mittels Ersetzen des normierten dimensionslosen Phasenwerts $\Phi_N$ in Gleichung 2 durch den degradationskompensierten Phasenwert $\psi_N$ und durch Entsprechendes Umformen der Gleichung 2 kann der normierte dimensionslose degradationskompensierte Phasenwert $\psi_N$ in einen dimensionsbehafteten degradationskompensierten Phasenwert $\psi$ umgerechnet werden.

Wird der degradationskompensierte Phasenwert $\psi$ anstelle des Phasenwerts $\Phi$ in den Kalibrations-Wertezusammenhang der Gleichung 1 eingesetzt, lässt sich aus dieser der gewünschte Quencher-Substanz-Partialdruck selbst mit einer degradierten Sensoranordnung 10 korrekt ermitteln und über die Datenleitung 59 an die Ausgabeeinrichtung 61 ausgeben.

Die obigen Wertezusammenhänge und Gleichungen sind im Datenspeicher 38 der Steuereinrichtung 36 hinterlegt. Die Steuereinrichtung 36 ist dazu ausgebildet, die obigen Rechenoperationen auszuführen und somit aus einem Erfassungswert der Sensoranordnung 10 einen korrekten Ergebniswert zu errechnen, und zwar selbst dann, wenn aufgrund von alterungsbedingter Degradation des Luminophors die einem Erfassungsvorgang zugrundeliegende Antwortcharakteristik sich von der der Erzeugung des Kalibrations-Wertezusammenhangs zugrundeliegenden Bezugs-Antwortcharakteristik unterscheidet, sogar erheblich unterscheidet.

Zur besseren Verständlichkeit dieses komplexen Themas sei die Handhabung der Ermittlung von Ergebniswerten aus degradationskompensierten Erfassungswerten noch einmal zusammengefasst:

Zunächst werden die für bauartgleiche Sensoranordnungen, also für einen Sensoranordnungstyp insgesamt, geltenden Gleichungen 4 und 8 durch Experimente mit Sensoranordnungen des betreffenden Typs in unterschiedlichen Degradationszuständen und mit unterschiedlichen Prüffluiden, vor allem mit unterschiedlichen Konzentrationen an Quencher-Substanz, für den Sensoranordnungstyp parametrisiert.

Die konkrete Sensoranordnung 10 wird kalibriert, d. h. die für die Sensoranordnung 10 zutreffenden Parameterwerte der Gleichung 1 werden anhand von wenigstens zwei Kalibrationsmessungen mit Prüffluiden mit betragsmäßig möglichst unterschiedlichem bekanntem Gehalt an Quencher-Substanz bestimmt.

Mit der Kalibration werden anhand der Erfassungswerte der Kalibrationsmessungen sowie anhand der bereits für den Sensoranordnungstyp parametrisierten Gleichung 4 mit Gleichung 5, umgeformt zu den Gleichungen 6 und 7, die Extremwerte $I_{max}$ und $I_{min}$ bestimmt.

Aus der durch Kalibration parametrisierten Gleichung 1 werden die Extremwerte $\Phi_{max}$ und $\Phi_{min}$ bestimmt.

Die Sensoranordnung 10 wird nun in den produktiven Erfassungsbetrieb genommen. Die erfassten Erfassungswerte werden durch die Gleichungen 2 und 3 in normierte dimensionslose Erfassungswerte umgerechnet.

Die normierten dimensionslosen Erfassungswertepaare ($\Phi_N$, $I_N$) werden als Argumente in Gleichung 8 verwendet. Mit der Gleichung 8 wird für jedes Erfassungswertepaar ($\Phi_N$, $I_N$) ein normierter dimensionsloser degradationskompensierter Erfassungswert ermittelt, der mit der entsprechend umgeformten Gleichung 2 in einen dimensionsbehafteten degradationskompensierten Erfassungswert umgerechnet wird.

Mit diesem dimensionsbehafteten degradationskompensierten Erfassungswert wird durch die parametrisierte Gleichung 1 der Ergebniswert ermittelt und von der Steuereinrichtung 36 ausgegeben.

Zusätzlich oder alternativ kann die Steuereinrichtung 36 anhand eines Vergleichs des degradationskompensierten Erfassungswerts mit dem tatsächlich erfassten Erfassungswert, sei es nun dimensionslos, dimensionslos normiert oder dimensionsbehaftet, oder/und anhand eines Vergleichs von auf Grundlage dieser beiden Erfassungswerte ermittelter Ergebniswerte den Degradationszustand der Sensoranordnung ermitteln und ausgeben.

Ausgehend von diesem Vergleich kann die Steuereinrichtung anhand der bekannten Degradationsverläufe, wie in den Figuren 4 bis 7 dargestellt, eine Abschätzung der Restlebensdauer der Sensoranordnung 10 ermitteln und ausgeben.

Ausgehend von diesem Vergleich kann die Steuereinrichtung zusätzlich oder alternativ entweder einen beruhend auf den tatsächlich erfassten Werte ermittelten Ergebniswert ausgeben, etwa dann, wenn ein Unterschiedsbetrag zwischen erfassten Werten und degradationskompensierten erfassten Werten kleiner als oder gleich wie eine vorbestimmte Akzeptanzschwelle ist, oder einen beruhend auf den degradationskompensierten erfassten Werten ermittelten Ergebniswert ausgeben etwa dann, wenn der Unterschiedsbetrag zwischen erfassten Werten und degradationskompensierten erfassten Werten größer als eine vorbestimmte Akzeptanzschwelle ist. Dabei wird mit einem beruhend auf den degradationskompensierten erfassten Werten ermittelten Ergebniswert vorzugsweise ein Hinweis ausgeben, dass dieser Wert nicht unmittelbar auf den tatsächlich erfassten Werten ermittelt wurde.

Die Steuereinrichtung 36, der Datenspeicher 38 und die Ausgabeeinrichtung 61 bilden eine Auswertevorrichtung 63.

## Patentansprüche

1. Verfahren für eine degradationskompensierte Auswertung von Erfassungssignalen einer nach dem Prinzip der Lumineszenzlöschung arbeitenden Sensoranordnung (10), welche Sensoranordnung (10) ein mit der Zeit degradierendes Luminophor (33), eine Anregungsstrahlungsquelle (46) sowie wenigstens einen optischen Sensor (50) aufweist, wobei das Luminophor (33) als Reaktion auf eine Bestrahlung mit einer vorgegebenen modulierten Anregungsstrahlung (E1) in Abhängigkeit vom Ausmaß einer Wechselwirkung des Luminophors (33) mit einer eine Lumineszenz des Luminophors (33) löschenden Quencher-Substanz (Q) gemäß einer sensoranordnungstypischen Antwortcharakteristik (62, 64, 66, 70, 72, 74) eine von dem wenigstens einen optischen Sensor (50) erfasste Antwortstrahlung (E2) abstrahlt, wobei die Sensoranordnung (10) als Erfassungssignale einen eine Intensität der Antwortstrahlung (E2) repräsentierenden erfassten Intensitätswert und einen einen Phasenunterschied der Antwortstrahlung (E2) bezüglich der Modulation der Anregungsstrahlung (E1) repräsentierenden erfassten Phasenwert ausgibt, **dadurch gekennzeichnet, dass** für eine erfolgte Erfassung einer Antwortstrahlung (E2) eine betragsmäßige Abweichung eines der erfassten Werte aus erfasstem Intensitätswert und erfasstem Phasenwert, welche Abweichung auf einer degradationsbasierten Änderung der Antwortcharakteristik (62, 64, 66, 70, 72, 74) zur Zeit der erfolgten Erfassung bezüglich einer Bezugs-Antwortcharakteristik (64, 70, 72, 74) beruht, die einer Kalibration der Sensoranordnung (10) zu Grunde liegt, nach Maßgabe sowohl des erfassten Intensitätswerts als auch des erfassten Phasenwerts betragsmäßig verringert und so ein degradationskompensierter erfasster Wert ermittelt wird, wobei ein auf die Quencher-Substanz (Q) bezogener Ergebniswert der erfolgten Erfassung nach Maßgabe eines vorbestimmten, unter Berücksichtigung der Bezugs-Antwortcharakteristik (64, 70, 72, 74) ermittelten Kalibrations-Wertezusammenhangs auf Grundlage des degradationskompensierten erfassten Werts bestimmt wird.

2. Verfahren nach Anspruch 1,
   **dadurch gekennzeichnet, dass** es den Schritt umfasst, den degradationskompensierten erfassten Wert auf Grundlage eines vorbestimmten Kompensations-Wertezusammenhangs mit Eingangsgrößen auf Grundlage des erfassten Intensitätswerts und des erfassten Phasenwerts zu ermitteln.

3. Verfahren nach Anspruch 1 oder 2,
   **dadurch gekennzeichnet, dass** es den Schritt umfasst, sowohl den erfassten Intensitätswert als auch den erfassten Phasenwert unter Verwendung von wenigstens einem die Sensoranordnung (10) charakterisierenden System parameter oder/und von wenigstens einem dem Erfassungsprozess entstammenden Prozessparameter in einen dimensionslosen erfassten Intensitätswert und in einen dimensionslosen erfassten Phasenwert zu transformieren, wobei dann der vorbestimmte Kompensations-Wertezusammenhang ein vorbestimmter dimensionsloser Kompensations-Wertezusammenhang ist, nach dessen Maßgabe mit Eingangsgrößen auf Grundlage des dimensionslosen erfassten Intensitätswerts und des dimensionslosen erfassten Phasenwerts, der degradationskompensierte erfasste Wert ermittelt wird.

**4.** Verfahren nach Anspruch 3,
**dadurch gekennzeichnet, dass** der degradationskompensierte erfasste Wert ein dimensionsloser degradationskompensierter erfasster Wert ist und dass der vorbestimmte Kalibrations-Wertezusammenhang ein dimensionsloser vorbestimmter Kalibrations-Wertezusammenhang ist, wobei der Ergebniswert nach Maßgabe des dimensionslosen Kalibrations-Wertezusammenhangs mit einer Eingangsgröße auf Grundlage des dimensionslosen degradationskompensierten Werts ermittelt wird.

**5.** Verfahren nach einem der vorhergehenden Ansprüche,
**dadurch gekennzeichnet, dass** der degradationskompensierte erfasste Wert ein degradationskompensierter erfasster Phasenwert ist.

**6.** Auswertevorrichtung (63), welche zur Ausführung des Verfahrens **nach einem der vorhergehenden Ansprüche** ausgebildet ist und somit ausgebildet ist zur degradationskompensierten Auswertung von Erfassungssignalen einer nach dem Prinzip der Lumineszenzlöschung arbeitenden Sensoranordnung (10) mit einem mit der Zeit degradierenden Luminophor (33), mit einer Anregungsstrahlungsquelle (46) sowie mit wenigstens einem optischen Sensor (50), wobei das Luminophor (33) als Reaktion auf eine Bestrahlung mit einer vorgegebenen modulierten Anregungsstrahlung (E1) in Abhängigkeit vom Ausmaß eines Kontakts des Luminophors (33) mit einer eine Lumineszenz des Luminophors (33) löschenden Quencher-Substanz (Q) gemäß einer sensoranordnungstypischen Antwortcharakteristik (62, 64, 66, 70, 72, 74) eine von dem wenigstens einen optischen Sensor (50) erfasste Antwortstrahlung (E2) abstrahlt, wobei die Auswertevorrichtung (63) einen Dateneingangskanal (54) aufweist, welcher dazu ausgebildet ist, von der Sensoranordnung (10) als Erfassungssignale den eine Intensität der Antwortstrahlung (E2) repräsentierenden erfassten Intensitätswert und den einen Phasenunterschied der Antwortstrahlung (E2) bezüglich der Modulation der Anregungsstrahlung (E1) repräsentierenden erfassten Phasenwert an eine Datenverarbeitungseinheit (39) der Auswertevorrichtung (63) zu übertragen, wobei die Datenverarbeitungseinheit (39) einen Datenspeicher (38) zur Speicherung von Daten und eine Recheneinheit (36) zur Verarbeitung von Daten aufweist, wobei in dem Datenspeicher (38) wenigstens der vorbestimmte, unter Berücksichtigung der Bezugs-Antwortcharakteristik (64, 70, 72, 74) ermittelte Kalibrations-Wertezusammenhang gespeichert ist, wobei die Auswertevorrichtung (63) dazu ausgebildet ist, den degradationskompensierten erfassten Wert aus erfasstem Intensitätswert und erfasstem Phasenwert nach Maßgabe sowohl des erfassten Intensitätswerts als auch des erfassten Phasenwerts zu ermitteln und den auf die Quencher-Substanz (Q) bezogenen Ergebniswert der erfolgten Erfassung nach Maßgabe des Kalibrations-Wertezusammenhangs auf Grundlage des degradationskompensierten erfassten Werts zu bestimmen und auszugeben.

**7.** Auswertevorrichtung (63) nach Anspruch 6,
**dadurch gekennzeichnet, dass** in dem Datenspeicher (38) auch der vorbestimmte Kompensations-Wertezusammenhang gespeichert ist, wobei die Recheneinheit (36) dazu ausgebildet ist, den degradationskompensierten erfassten Wert nach Maßgabe des vorbestimmten Kompensations-Wertezusammenhangs mit Eingangsgrößen auf Grundlage des erfassten Intensitätswerts und des erfassten Phasenwerts zu ermitteln.

**8.** Auswertevorrichtung (63) nach Anspruch 6 oder 7,
**dadurch gekennzeichnet, dass** die Auswertevorrichtung (63) dazu ausgebildet ist, sowohl den erfassten Intensitätswert als auch den erfassten Phasenwert unter Verwendung von wenigstens einem die Sensoranordnung (10) charakterisierenden Systemparameter oder/und von wenigstens einem dem Erfassungsprozess entstammenden Prozessparameter in einen dimensionslosen erfassten Intensitätswert und in einen dimensionslosen erfassten Phasenwert zu transformieren, wobei der vorbestimmte Kompensations-Wertezusammenhang ein vorbestimmter dimensionsloser Kompensations-Wertezusammenhang ist, nach dessen Maßgabe mit Eingangsgrößen auf Grundlage des dimensionslosen erfassten Intensitätswerts und des dimensionslosen erfassten Phasenwerts, der degradationskompensierte erfasste Wert ermittelt wird.

**9.** Auswertevorrichtung (63) nach Anspruch 8,
**dadurch gekennzeichnet, dass** der degradationskompensierte erfasste Wert ein dimensionsloser degradationskompensierter erfasster Wert ist und dass der vorbestimmte Kalibrations-Wertezusammenhang ein dimensionsloser vorbestimmter Kalibrations-Wertezusammenhang ist, wobei die Auswertevorrichtung (63) dazu ausgebildet ist, den Ergebniswert nach Maßgabe des dimensionslosen Kalibrations-Wertezusammenhangs mit einer Eingangsgröße auf Grundlage des dimensionslosen degradationskompensierten Werts zu ermitteln.

**10.** Auswertevorrichtung (63) nach einem der Ansprüche 6 bis 9,
**dadurch gekennzeichnet, dass** der degradationskompensierte erfasste Wert ein degradationskompensierter erfasster Phasenwert ist.

11. Messanordnung umfassend eine Auswertevorrichtung (63) nach einem der Ansprüche 6 bis 10 und eine Sensoranordnung (10) mit einem mit der Zeit degradierenden Luminophor (33), mit einer Anregungsstrahlungsquelle (46) sowie mit wenigstens einem optischen Sensor (50), wobei das Luminophor (33) als Reaktion auf eine Bestrahlung mit einer vorgegebenen modulierten Anregungsstrahlung (E1) in Abhängigkeit vom Ausmaß eines Kontakts des Luminophors (33) mit einer eine Lumineszenz des Luminophors (33) löschenden Quencher-Substanz (Q) gemäß einer sensoranordnungstypischen Antwortcharakteristik (62, 64, 66, 70, 72, 74) eine von dem wenigstens einen optischen Sensor (50) erfasste Antwortstrahlung (E2) abstrahlt.

## Claims

1. A method for degradation-compensated evaluation of detection signals of a sensor arrangement (10) operating on the principle of luminescence quenching, which sensor arrangement (10) comprises a luminophore (33) that degrades over time, an excitation radiation source (46), and at least one optical sensor (50), the luminophore (33) radiating, in accordance with a response characteristic (62, 64, 66, 70, 72, 74) typical of the sensor arrangement, in reaction to irradiation with a predefined modulated excitation radiation (E1) and as a function of the extent of an interaction of the luminophore (33) with a quencher substance (Q) that quenches the luminescence of the luminophore (33), a response radiation (E2) detected by the at least one optical sensor (50); the sensor arrangement (10) outputting, as detection signals, a detected intensity value representing an intensity of the response radiation (E2) and a detected phase value representing a phase difference of the response radiation (E2) with respect to the modulation of the excitation radiation (E1);
**characterized in that** for an accomplished detection of a response radiation (E2), a quantitative deviation of one of the detected values, from among a detected intensity value and detected phase value, being quantitatively decreased in accordance both with the detected intensity value and with the detected phase value, the deviation being based on a degradation-based change in the response characteristic (62, 64, 66, 70, 72, 74) at the time of the accomplished detection, with respect to a reference response characteristic (64, 70, 72, 74) which is a basis of a calibration of the sensor arrangement (10), a degradation-compensated detected value thus being identified; a result value of the accomplished detection, referred to the quencher substance (Q), being determined on the basis of the degradation-compensated detected value in accordance with a predetermined calibration value correlation identified in consideration of the reference response characteristic (64, 70, 72, 74).

2. The method according to Claim 1,
**characterized in that** it encompasses the step of identifying the degradation-compensated detected value on the basis of a predetermined compensation value correlation with input variables on the basis of the detected intensity value and the detected phase value.

3. The method according to Claim 1 or 2,
**characterized in that** it encompasses the step of transforming both the detected intensity value and the detected phase value, using at least one system parameter characterizing the sensor arrangement (10) and/or at least one process parameter deriving from the detection process, into a dimensionless detected intensity value and into a dimensionless detected phase value, the predetermined compensation value correlation then being a predetermined dimensionless compensation value correlation in accordance with which, with input variables on the basis of the dimensionless detected intensity value and the dimensionless detected phase value, the degradation-compensated detected value is identified.

4. The method according to Claim 3,
**characterized in that** the degradation-compensated detected value is a dimensionless degradation-compensated detected value; and the predetermined calibration value correlation is a dimensionless predetermined calibration value correlation, the result value being identified in accordance with the dimensionless calibration value correlation with an input variable on the basis of the dimensionless degradation-compensated value.

5. The method according to one of the preceding claims,
**characterized in that** the degradation-compensated detected value is a degradation-compensated detected phase value.

6. An evaluation apparatus (63) that is embodied to execute the method **according to one of the preceding claims** and is thus embodied for degradation-compensated evaluation of detection signals of a sensor arrangement (10) that operates according to the principle of luminescence quenching and has a luminophore (33) that degrades over time, has an excitation radiation source (46), and has at least one optical sensor (50); the luminophore (33) radiating, in accordance with a response characteristic (62, 64, 66, 70, 72, 74) typical of the sensor arrangement, in reaction to irradiation with a predefined modulated excitation radiation (E1) and as a function of the extent of a contact of the luminophore (33) with a quencher substance (Q) that quenches the luminescence of the luminophore (33), a response radiation (E2) detected by the at least one optical sensor (50); the evaluation apparatus (63) comprising a data input channel (54) that is embodied to transfer from the sensor arrangement (10) to a data processing unit (39) of the evaluation apparatus (63), as detection signals, the detected intensity value representing the intensity of the response radiation (E2) and the detected phase value representing a phase difference of the response radiation (E2) with respect to the modulation of the excitation radiation (E1); the data processing unit (39) comprising a data memory (38) for storing data and a computation unit (36) for processing data; at least the predetermined calibration value correlation identified in consideration of the reference response characteristic (64, 70, 72, 74) being stored in the data memory (38); the evaluation apparatus (63) being embodied to ascertain the degradation-compensated detected value from the detected intensity value and detected phase value in accordance with both the detected intensity value and the detected phase value, and to determine and output the result value, referred to the quencher substance (Q), of the accomplished detection in accordance with the calibration value correlation on the basis of the degradation-compensated detected value.

7. The evaluation apparatus (63) according to Claim 6,
**characterized in that** the predetermined compensation value correlation is also stored in the data memory (38), the computation unit (36) being embodied to identify the degradation-compensated detected value in accordance with the predetermined compensation value correlation with input values on the basis of the detected intensity value and of the detected phase value.

8. The evaluation apparatus (63) according to Claim 6 or 7,
**characterized in that** the evaluation apparatus (63) is embodied to transform both the detected intensity value and the detected phase value, utilizing at least one system parameter characterizing the sensor arrangement (10) and/or at least one process parameter deriving from the detection process, into a dimensionless detected intensity value and into a dimensionless detected phase value, the predetermined compensation value correlation being a predetermined dimensionless compensation value correlation in accordance with which, with input variables on the basis of the dimensionless detected intensity value and the dimensionless detected phase value, the degradation-compensated detected value is identified.

9. The evaluation apparatus (63) according to Claim 8,
**characterized in that** the degradation-compensated detected value is a dimensionless degradation-compensated detected value; and the predetermined calibration value correlation is a dimensionless predetermined calibration value correlation, the evaluation apparatus (63) being embodied to ascertain the result value in accordance with the dimensionless calibration value correlation with an input variable on the basis of the dimensionless degradation-compensated value.

10. The evaluation apparatus (63) according to one of Claims 6 to 9,
**characterized in that** the degradation-compensated detected value is a degradation-compensated detected phase value.

11. A measurement arrangement encompassing an evaluation apparatus (63) according to Claims 6 to 10 and a sensor arrangement (10) having a luminophore (33) that degrades over time, having an excitation radiation source (46), and having at least one optical sensor (50); the luminophore (33) radiating, in accordance with a response characteristic (62, 64, 66, 70, 72, 74) typical of the sensor arrangement, in reaction to irradiation with a predefined modulated excitation radiation (E1) and as a function of the extent of a contact of the luminophore (33) with a quencher substance (Q) that quenches the luminescence of the luminophore (33), a response radiation (E2) detected by the at least one optical sensor (50).

## Revendications

1. Procédé pour une évaluation compensée en termes de dégradation de signaux de détection d'un agencement de capteurs (10) fonctionnant selon le principe de l'extinction de la luminescence, lequel agencement de capteurs (10) présente un luminophore (33) se dégradant avec le temps, une source de rayonnement d'excitation (46) ainsi qu'au moins un capteur optique (50), dans lequel le luminophore (33), en réacton à une irradiation avec un rayonnement d'excitation modulé prédéterminé (E1), en fonction du degré d'interaction du luminophore (33) avec une substance d'extinction (Q) qui éteint la luminescence du luminophore (33), selon une caractéristique de réponse (62, 64, 66, 70, 72, 74) typique de l'agencement de capteurs émet un rayonnement de réponse (E2) détecté par le au moins un capteur optique (50), l'agencement de capteurs (10) émettant comme signaux de détection une valeur d'intensité détectée représentant une intensité du rayonnement de réponse (E2) et une valeur de phase détectée représentant une différence de phase du rayonnement de réponse (E2) par rapport à la modulation du rayonnement d'excitation (E1), **caractérisé en ce que,** pour une détection effectuée d'un rayonnement de réponse (E2), un écart de valeur de l'une des valeurs détectées de la valeur d'intensité détectée et de la valeur de phase détectée, lequel écart est basé sur une modification basée sur la dégradation de la caractéristique de réponse (62, 64, 66, 70, 72, 74) au moment de la détection effectuée par rapport à une caractéristique de réponse de référence (64, 70, 72, 74), qui est à la base d'un étalonnage de l'agencement de capteurs (10), en fonction aussi bien de la valeur d'intensité détectée que de la valeur de phase détectée, de manière à déterminer une valeur détectée compensée en termes de dégradation, dans lequel une valeur de résultat de la détection effectuée, rapportée à la substance d'extinction (Q), est déterminée en fonction d'une relation de valeurs d'étalonnage prédéterminée, déterminée en tenant compte de la caractéristique de réponse de référence (64, 70, 72, 74), sur la base de la valeur détectée compensée en termes de dégradation.

2.  Procédé selon la revendication 1,
    **caractérisé en ce qu'**il comprend l'étape consistant à déterminer la valeur détectée compensée en dégradation sur la base d'une relation prédéterminée de valeurs de compensation avec des entrées basées sur la valeur d'intensité détectée et la valeur de phase détectée.

3.  Procédé selon la revendication 1 ou 2,
    **caractérisé en ce qu'**il comprend l'étape consistant à transformer aussi bien la valeur d'intensité détectée que la valeur de phase détectée en une valeur d'intensité détectée sans dimension et en une valeur de phase détectée sans dimension en utilisant au moins un paramètre de système caractérisant l'agencement de capteurs (10) ou/et au moins un paramètre de processus provenant du processus de détection, la relation prédéterminée de valeur de compensation étant une relation prédéterminée de valeur de compensation sans dimension, selon laquelle la valeur détectée compensée en dégradation est déterminée avec des grandeurs d'entrée sur la base de la valeur d'intensité détectée sans dimension et de la valeur de phase détectée sans dimension.

4.  Procédé selon la revendication 3,
    **caractérisé en ce que** la valeur détectée compensée en dégradation est une valeur détectée compensée en dégradation sans dimension et **en ce que** la relation prédéterminée de valeurs d'étalonnage est une relation prédéterminée de valeurs d'étalonnage sans dimension, la valeur de résultat étant déterminée en fonction de la relation prédéterminée de valeurs d'étalonnage sans dimension avec une grandeur d'entrée sur la base de la valeur compensée en dégradation sans dimension.

5.  Procédé selon l'une des revendications précédentes,
    **caractérisé en ce que** la valeur détectée compensée en termes de dégradation est une valeur de phase détectée compensée en termes de dégradation.

6.  Dispositif d'évaluation (63) qui est conçu pour la mise en œuvre du procédé **selon l'une des revendications précédentes** et qui est ainsi conçu pour l'évaluation compensée en dégradation de signaux de détection d'un agencement de capteurs (10) fonctionnant selon le principe de l'extinction de la luminescence, avec un luminophore (33) se dégradant avec le temps, avec une source de rayonnement d'excitation (46) ainsi qu'avec au moins un capteur optique (50), dans lequel le luminophore (33) en réacton à une irradiation par un rayonnement d'excitation modulé prédéterminé (E1) en fonction du degré de contact du luminophore (33) avec une substance d'extinction (Q) qui éteint la luminescence du luminophore (33) selon une caractéristique de réponse typique d'un dispositif de détection (62, 64, 66, 70, 72, 74) émet un rayonnement de réponse (E2) détecté par le au moins un capteur optique (50), le dispositif d'évaluation (63) présentant un canal d'entrée de données (54) qui est conçu pour transmettre à une unité de traitement de données (39) du dispositif d'évaluation (63), à partir de l'agencement de capteurs (10), en tant que signaux de détection, la valeur d'intensité détectée représentant une intensité du rayonnement de réponse (E2) et la valeur de phase détectée représentant une différence de phase du rayonnement de réponse (E2) par rapport à la modulation du rayonnement d'excitation (E1), l'unité de traitement de données (39) présentant une mémoire de données (38) pour le stockage de données et une unité de calcul (36) pour le traitement de données, au moins la relation de valeurs d'étalonnage déterminée en tenant compte de la caractéristique de réponse de référence (64, 70, 72, 74) étant mémorisée dans la mémoire de données (38), le dispositif d'évaluation (63) étant conçu pour déterminer la valeur détectée compensée en dégradation parmi la valeur d'intensité détectée et la valeur de phase détectée en fonction aussi bien de la valeur d'intensité détectée que de la valeur de phase détectée, et pour déterminer et délivrer la valeur de résultat rapportée à la substance d'extinction (Q) de la détection effectuée en fonction de la relation de valeurs d'étalonnage sur la base de la valeur détectée compensée en dégradation.

7. Dispositif d'évaluation (63) selon la revendication 6,
   **caractérisé en ce que** la relation prédéterminée de valeurs de compensation est également mémorisée dans la mémoire de données (38), l'unité de calcul (36) étant conçu pour déterminer la valeur détectée compensée en dégradation en fonction de la relation prédéterminée de valeurs de compensation avec des grandeurs d'entrée sur la base de la valeur d'intensité détectée et de la valeur de phase détectée.

8. Dispositif d'évaluation (63) selon la revendication 6 ou 7,
   **caractérisé en ce que** le dispositif d'évaluation (63) est conçu pour transformer aussi bien la valeur d'intensité détectée que la valeur de phase détectée en une valeur d'intensité détectée sans dimension et en une valeur de phase détectée sans dimension en utilisant au moins un paramètre de système caractérisant l'agencement de capteurs (10) ou/et au moins un paramètre de processus provenant du processus de détection, la relation prédéterminée de valeurs de compensation étant une relation prédéterminée de valeurs de compensation sans dimension, selon laquelle la valeur détectée compensée en dégradation est déterminée avec des grandeurs d'entrée sur la base de la valeur d'intensité détectée sans dimension et de la valeur de phase détectée sans dimension.

9. Dispositif d'évaluation (63) selon la revendication 8,
   **caractérisé en ce que** la valeur détectée compensée en dégradation est une valeur détectée compensée en dégradation sans dimension et **en ce que** la relation prédéterminée de valeurs d'étalonnage est une relation prédéterminée de valeurs d'étalonnage sans dimension, le dispositif d'évaluation (63) étant conçu pour déterminer la valeur de résultat en fonction de la relation prédéterminée de valeurs d'étalonnage sans dimension avec une grandeur d'entrée sur la base de la valeur compensée en dégradation sans dimension.

10. Dispositif d'évaluation (63) selon l'une des revendications 6 à 9,
    **caractérisé en ce que** la valeur détectée compensée en dégradation est une valeur de phase détectée compensée en dégradation.

11. Agencement de mesure comprenant un dispositif d'évaluation (63) selon l'une des revendications 6 à 10 et un agencement de capteurs (10) avec un luminophore (33) se dégradant avec le temps, avec une source de rayonnement d'excitation (46) ainsi qu'avec au moins un capteur optique (50), dans lequel le luminophore (33) émet un rayonnement de réponse (E2) détecté par ledit au moins un capteur optique (50) en réacton à une irradiation par un rayonnement d'excitation modulé prédéterminé (E1) en fonction du degré d'un contact du luminophore (33) avec une substance d'extinction (Q) qui éteint la luminescence du luminophore (33) selon une caractéristique de réponse (62, 64, 66, 70, 72, 74) typique de l'agencement de capteurs.

Fig. 1

EP 3 794 334 B1

Fig. 2

Fig. 3

Fig. 4

Fig. 5

Fig. 6

Fig. 7

Fig. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2887054 A1
- GB 2496657 A
- GB 2479183 A
- GB 2380790 A
- WO 2007066126 A1
- WO 2004077035 A1
- DE 19709377 A1
- JP 2010133725 A
- US 7926322 B1
- US 5030420 A
- US 20110184259 A1
- US 5060505 A
- US 5887048 A
- US 6205272 B1
- US 20060018045 A1
- US 20080085217 A1